(19) Europäisches Patentamt
European Patent Office
Office européen des brevets

(11) **EP 4 304 462 B1**

(12) **EUROPEAN PATENT SPECIFICATION**

(45) Date of publication and mention
of the grant of the patent:
**01.04.2026 Bulletin 2026/14**

(21) Application number: **22726213.6**

(22) Date of filing: **19.04.2022**

(51) International Patent Classification (IPC):
*A61B 5/02* $^{(2006.01)}$         *A61B 5/021* $^{(2006.01)}$
*A61B 5/1455* $^{(2006.01)}$       *A61B 5/00* $^{(2006.01)}$

(52) Cooperative Patent Classification (CPC):
**A61B 5/02028; A61B 5/021; A61B 5/14551;
A61B 5/7257**

(86) International application number:
**PCT/US2022/025386**

(87) International publication number:
**WO 2022/231888 (03.11.2022 Gazette 2022/44)**

(54) **SYSTEM AND METHOD FOR AUTOREGULATION DATA DETERMINATION**

SYSTEM UND VERFAHREN ZUR AUTOREGULATIONSDATENBESTIMMUNG

SYSTÈME ET PROCÉDÉ DE DÉTERMINATION DE DONNÉES DE RÉGULATION AUTOMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2021 US 202163181108 P**

(43) Date of publication of application:
**17.01.2024 Bulletin 2024/03**

(73) Proprietor: **Becton, Dickinson and Company
Franklin Lakes, NJ 07417-1880 (US)**

(72) Inventors:
• **BENNI, Paul, B.**
  **Irvine, CA 92614 (US)**
• **ALBANESE, Antonio**
  **Irvine, CA 92614 (US)**
• **ALATHUR RANGARAJAN, Anusha**
  **Irvine, CA 92614 (US)**
• **AGUIRRE, Andres, S.**
  **Irvine, CA 92614 (US)**
• **SCHNEIDER, Brennan, Michael**
  **Irvine, CA 92614 (US)**

(74) Representative: **Venner, Julia Ann et al
Kilburn & Strode LLP
Lacon London
84 Theobalds Road
London WC1X 8NL (GB)**

(56) References cited:
US-A1- 2017 105 672     US-A1- 2020 129 076
US-A1- 2020 383 616     US-A1- 2021 016 136
US-B2- 10 987 065

**Description**

This application claims priority based on United States Provisional Patent Application Serial No. 63/181,108, filed April 28, 2021 and entitled SYSTEM AND METHOD FOR

AUTOREGULATION DATA DETERMINATION.

BACKGROUND OF THE INVENTION

1. Technical Field

The present disclosure relates to medical apparatus and methods in general, and to medical apparatus and methods for measuring and/or monitoring autoregulation in particular.

2. Background Information

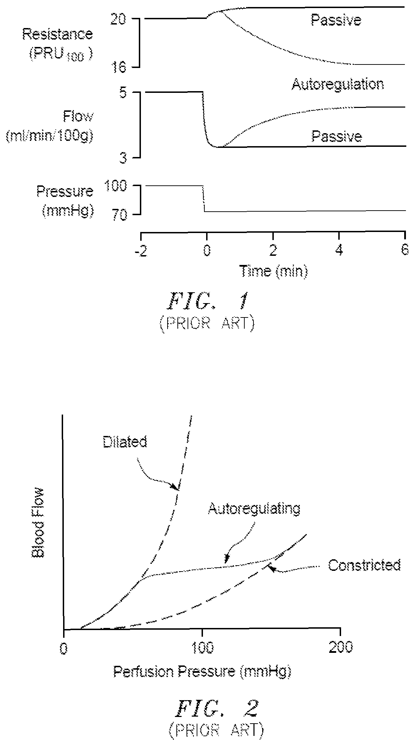

Autoregulation is a process in mammals that aims to maintain adequate and stable (e.g., "constant") blood flow to organs (e.g., brain, heart, kidneys, etc.) for a range of perfusion pressures. While most systems of the body show some degree of autoregulation, the brain is very sensitive to overperfusion as well as underperfusion. FIG. 1 shows the effects of suddenly reducing perfusion pressure from 100 to 70 mmHg. In a passive vascular bed (i.e., poor autoregulation), this sudden drop in pressure will result in a rapid and sustained fall in blood flow. With autoregulation, vascular resistance is increased, in an effort to return to nominal flow. The range that vascular resistance can vary has limitations, however. Arterial blood vessels can reach a point of maximum dilation due to a vasodilator drug or other cause, in which vascular reactivity (i.e., the ability to change vascular resistance) becomes passive. In a passive state, a change in blood pressure may result in a change in blood flow. If the blood flow decreases sufficiently, inadequate perfusion and resultant ischemia within the organ may occur. Conversely, arterial blood vessels can reach a state of maximum constriction, in which vascular reactivity also becomes passive. Increased blood pressure can result in excessive flow to the organ; e.g., see FIG. 2.

Different organs display varying degrees of autoregulatory behavior. The renal, cerebral, and coronary circulations typically show excellent autoregulation, whereas skeletal muscle and splanchnic circulations show moderate autoregulation. The cutaneous circulation shows little or no autoregulatory capacity.

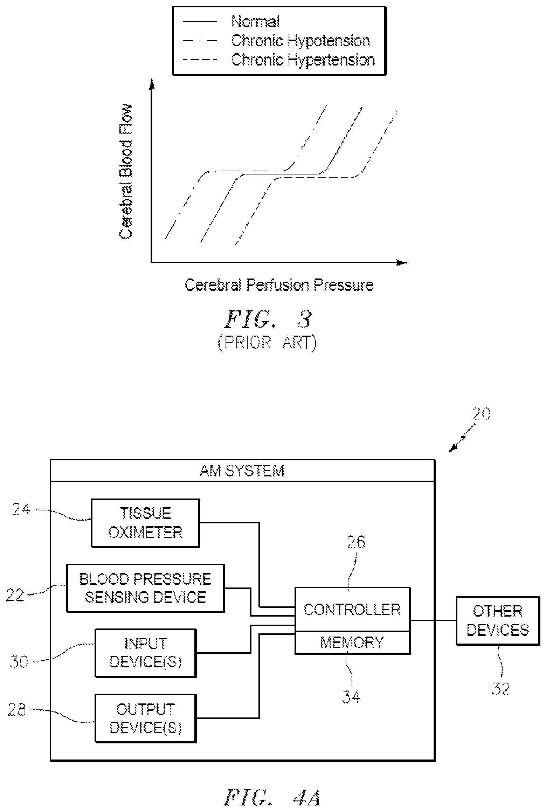

A plurality of factors (e.g., a hardening of the arteries that occurs with advancing age) can change the characteristics of a vascular reactivity response, and these factors can in turn change relevant autoregulation characteristics. Hence, the autoregulation range of blood flow due to changing blood pressure can vary between subjects and cannot be assumed to be constant. FIG. 3 illustrates how a cerebral autoregulation curve can shift due to chronic hypertension and hypotension. Methods and apparatus for determining whether a particular subject's autoregulation is functioning, and the potential range to manage blood pressure variability, would be a great help to a clinician.

US 2020/383616 describes an autoregulation system and method using tissue oximetry and blood pressure. US 2020/129076 describes a system and method for identifying anomalous autoregulation state values. US 2017/105672 describes a system and method for identifying autoregulation zones. US 2021/016136 describes a fitness management method, device and computer readable storage medium.

What is needed is an apparatus and method for monitoring autoregulation that is an improvement over those known in the prior art, including one that identifies and accounts for factors that may confound an autoregulation determination or measurement.

SUMMARY OF THE INVENTION

Aspects of the presently claimed invention are set out in the independent claims. Particular embodiments of these aspects are set out in the dependent claims. Any subject matter contained herein that does not fall within the scope of the appended claims is considered as being useful for understanding the invention.

According to an aspect of the present invention there is provided a method for providing information regarding a subject's autoregulation function state, comprising:

continuously sensing a tissue region of a subject with a tissue oximeter during a period of time, the sensing producing first signals representative of at least one tissue oxygenation parameter;
continuously measuring a blood pressure level of the subject during the period of time using a blood pressure sensing device, the measuring producing second signals representative of the blood pressure level of the subject;
evaluating the at least one tissue oxygenation parameter using the first signals and the blood pressure level of the subject using the second signals, relative to one another, wherein the step of evaluating the at least one tissue oxygenation parameter using the first signals and the blood pressure level of the subject using the second signals, relative to one another includes:

determining frequency domain tissue oxygen parameter values by performing a first frequency domain transformation of the first signals;
determining frequency domain blood pressure values by performing a second frequency domain transformation of the second signals; and
determining coherence (COHZ) values indicative of the subject's autoregulation state using the frequency domain tissue oxygen parameter values and the frequency domain blood pressure values;
producing a recent profile of autoregulation data representative of the evaluated said at least one tissue oxygenation parameter and said blood pressure level relative to one another using said first signals and said second signals from a recent portion of the period of time;
producing a historical profile of autoregulation data representative of the evaluated said at least one tissue oxygenation parameter and said blood pressure level relative to one another using said first signals and said second signals from a historical portion of the period of time, wherein the historical portion of the period of time is longer than the recent portion of the period of time, and the historical profile of autoregulation data is independent of the recent profile of autoregulation data, wherein the historical portion of the period of time extends less than an entirety of the period of time by an amount substantially equal to the recent portion of the period of time; and
displaying the historical profile and the recent profile together;
wherein both the recent profile of autoregulation data and the historical profile of autoregulation data include the COHZ values as a function of the blood pressure level.

According to another aspect of the present invention there is provided an apparatus for providing information regarding a subject's autoregulation function state, comprising:

a near infra-red spectroscopy (NIRS) tissue oximeter, configured to continuously sense a tissue region of the subject;
a blood pressure sensing device, configured to continuously measure a blood pressure level of the subject; and
a controller in communication with the NIRS tissue oximeter and the blood pressure sensing device, the controller including at least one processor and a memory device configured to store instructions, the stored instructions when executed cause the controller to:

control the NIRS tissue oximeter to continuously sense a tissue region of the subject during a period of time, and to produce first signals representative of at least one tissue oxygenation parameter;
control the blood pressure sensing device to continuously measure a blood pressure level of the subject during the period of time, and to produce second signals representative of the measured blood pressure level of the subject;
evaluate the at least one tissue oxygenation parameter using the first signals and the blood pressure level of the subject using the second signals, relative to one another, wherein evaluating the at least one tissue oxygenation parameter using the first signals and the blood pressure level of the subject using the second signals, relative to one another includes:

determining frequency domain tissue oxygen parameter values by performing a first frequency domain transformation of the first signals;
determining frequency domain blood pressure values by performing a second frequency domain transformation of the second signals; and
determining coherence (COHZ) values indicative of the subject's autoregulation state using the frequency domain tissue oxygen parameter values and the frequency domain blood pressure values;
produce a recent profile of autoregulation data representative of the evaluated said at least one tissue oxygenation parameter and said blood pressure level relative to one another using said first signals and said second signals from a recent portion of the period of time;
produce a historical profile of autoregulation data representative of the evaluated said at least one tissue oxygenation parameter and said blood pressure level relative to one another using said first signals and said second signals from a historical portion of the period of time, wherein the historical portion of the period of time is longer than the recent portion of the period of time, and the historical profile of autoregulation data is independent of the recent profile of autoregulation data, wherein the historical portion of the period of time extends less than an entirety of the period of time by an amount substantially equal to the recent portion of the period of time; and

display the historical profile and the recent profile together;

wherein both the recent profile of autoregulation data and the historical profile of autoregulation data include the COHZ values as a function of the blood pressure level.

According to another aspect of the present invention there is provided a non-transitory computer-readable medium containing computer program instructions, wherein the computer program instructions are executable by the at least one computer processor to perform a method of providing information regarding a subject's autoregulation function state, the method comprising:

controlling a near infra-red spectroscopy (NIRS) tissue oximeter to continuously sense a tissue region of a subject during a period of time, the sensing producing first signals representative of at least one tissue oxygenation parameter;

controlling a blood pressure sensing device to continuously measure a blood pressure level of the subject during the period of time, the measuring producing second signals representative of the blood pressure level of the subject;

evaluating the at least one tissue oxygenation parameter using the first signals and the blood pressure level of the subject using the second signals, relative to one another, wherein the step of evaluating the at least one tissue oxygenation parameter using the first signals and the blood pressure level of the subject using the second signals, relative to one another includes:

determining frequency domain tissue oxygen parameter values by performing a first frequency domain transformation of the first signals;

determining frequency domain blood pressure values by performing a second frequency domain transformation of the second signals; and

determining coherence (COHZ) values indicative of the subject's autoregulation state using the frequency domain tissue oxygen parameter values and the frequency domain blood pressure values;

producing a recent profile of autoregulation data representative of the evaluated said at least one tissue oxygenation parameter and said blood pressure level relative to one another using said first signals and said second signals from a recent portion of the period of time;

producing a historical profile of autoregulation data representative of the evaluated said at least one tissue oxygenation parameter and said blood pressure level relative to one another using said first signals and said second signals from a historical portion of the period of time, wherein the historical portion of the period of time is longer than the recent portion of the period of time, and the historical profile of autoregulation data is independent of the recent profile of autoregulation data, wherein the historical portion of the period of time extends less than an entirety of the period of time by an amount substantially equal to the recent portion of the period of time; and

displaying the historical profile and the recent profile together;

wherein both the recent profile of autoregulation data and the historical profile of autoregulation data include the COHZ values as a function of the blood pressure level.

## SUMMARY OF THE DISCLOSURE

According to an aspect of the present disclosure useful for understanding the presently claimed invention, a method for providing information regarding a subject's autoregulation function state is provided. The method includes: a) continuously sensing a tissue region of a subject with a tissue oximeter during a period of time, the sensing producing first signals representative of at least one tissue oxygenation parameter; b) continuously measuring a blood pressure level of the subject during the period of time using a blood pressure sensing device, the measuring producing second signals representative of the blood pressure level of the subject; c) evaluating the at least one tissue oxygenation parameter using the first signals and the blood pressure level of the subject using the second signals, relative to one another; d) producing a recent profile of autoregulation data representative of the evaluated at least one tissue oxygenation parameter and the blood pressure level relative to one another using the first and second signals from a recent portion of the period of time; e) producing a historical profile of autoregulation data representative of the evaluated at least one tissue oxygenation parameter and the blood pressure level relative to one another using the first and second signals from a historical portion of the period of time, wherein the historical portion of the period of time is longer than the recent portion of the period of time, and the historical profile of autoregulation data is independent of the recent profile of autoregulation data.

In any of the aspects or embodiments described above and herein, the historical portion of the period of time may extend an entirety of the period of time.

According to the present invention, the historical portion of the period of time extends less than an entirety of the period of time by an amount substantially equal to the recent portion of the period of time.

In any of the aspects or embodiments described above and herein, the period of time may extend between a first point in time T1 and a second point in time T2, wherein the second point in time is later that the first point in time T1. The recent profile of autoregulation data may be produced using the first and second signals from the recent portion of the period of time, the recent portion of the period of time extending between the second point in time T2 and a third point in time T3, wherein the third point in time is earlier than second point in time T2 and later than the first point in time T1. The historical profile of autoregulation data may be produced using the first and second signals from the historical portion of the period of time, the historical portion of the period of time extending between the first point in time T1 and the third point in time T3.

In any of the aspects or embodiments described above and herein, the period of time may extend between the first point in time T1 and a new second point in time NT2, and the new second point in time NT2 is later that the second point in time T2. The method may further include: a) updating the historical profile using the recent profile of autoregulation data; and b) producing a new recent profile of autoregulation data representative of the evaluated at least one tissue oxygenation parameter and the blood pressure level relative to one another using the first and second signals from a new recent portion of the period of time, the new recent portion of the period of time extending between the new second point in time NT2 and the second point in time T2.

According to the present invention, the step of evaluating the at least one tissue oxygenation parameter using the first signals and the blood pressure level of the subject using the second signals, relative to one another includes: a) determining frequency domain tissue oxygen parameter values by performing a first frequency domain transformation of the first signals; b) determining frequency domain blood pressure values by performing a second frequency domain transformation of the second signals; and c) determining coherence (COHZ) values indicative of the subject's autoregulation state using the frequency domain tissue oxygen parameter values and the frequency domain blood pressure values.

According to the present invention, both the recent profile of autoregulation data and the historical profile of autoregulation data include the COHZ values as a function of the blood pressure level.

According to the present invention, the method further includes displaying the historical profile and the recent profile together.

According to another aspect of the present disclosure useful for understanding the presently claimed invention, an apparatus for providing information regarding a subject's autoregulation function state is provided. The apparatus includes a near infra-red spectroscopy (NIRS) tissue oximeter, a blood pressure sensing device, and a controller. The NIRS tissue oximeter is configured to continuously sense a tissue region of the subject. The blood pressure sensing device is configured to continuously measure a blood pressure level of the subject. The controller is in communication with the NIRS tissue oximeter and the blood pressure sensing device. The controller includes at least one processor and a memory device configured to store instructions. The stored instructions when executed cause the controller to: a) control the NIRS tissue oximeter to continuously sense a tissue region of the subject during a period of time, and to produce first signals representative of at least one tissue oxygenation parameter; b) control the blood pressure sensing device to continuously measure a blood pressure level of the subject during the period of time, and to produce second signals representative of the measured blood pressure level of the subject; c) evaluate the at least one tissue oxygenation parameter using the first signals and the blood pressure level of the subject using the second signals, relative to one another; d) produce a recent profile of autoregulation data representative of the evaluated at least one tissue oxygenation parameter and the blood pressure level relative to one another using the first and second signals from a recent portion of the period of time; e) produce a historical profile of autoregulation data representative of the evaluated at least one tissue oxygenation parameter and the blood pressure level relative to one another using the first signals and second signals from a historical portion of the period of time. The historical portion of the period of time is longer than the recent portion of the period of time, and the historical profile of autoregulation data is independent of the recent profile of autoregulation data.

According to another aspect of the present disclosure useful for understanding the presently claimed invention, a method for providing information regarding a subject's cerebral autoregulation function state is provided. The method includes: a) continuously sensing at least a portion of a left hemisphere portion of a subject's brain with a tissue oximeter during a period of time, the sensing producing first signals representative of at least one tissue oxygenation parameter within the left hemisphere; b) continuously sensing at least a portion of a right hemisphere portion of a subject's brain with the tissue oximeter during the period of time, the sensing producing second signals representative of the least one tissue oxygenation parameter within the right hemisphere; c) continuously measuring a blood pressure level of the subject during the period of time using a blood pressure sensing device, the measuring producing third signals representative of the blood pressure level of the subject; d) producing a left hemisphere autoregulation data profile using the first and third signals; e) producing a right hemisphere autoregulation data profile using the second and third signals; f) producing a combined sides autoregulation profile using the left hemisphere autoregulation data profile and the right hemisphere autoregulation data profile.

In any of the aspects or embodiments described above and herein, the step of producing the left hemisphere autoregulation data profile may include evaluating the at least one tissue oxygenation parameter using the first signals and the blood pressure level of the subject using the third signals, relative to one another, and the step of producing the right hemisphere autoregulation data profile includes evaluating the at least one tissue oxygenation parameter using the second signals and the blood pressure level of the subject using the third signals, relative to one another.

In any of the aspects or embodiments described above and herein, the step of evaluating the at least one tissue oxygenation parameter using the first signals and the blood pressure level of the subject using the third signals, relative to one another may include: a) determining left side frequency domain tissue oxygen parameter values by performing a frequency domain transformation of the first signals; b) determining frequency domain blood pressure values by performing a frequency domain transformation of the third signals; and c) determining left hemisphere coherence (COHZ) values indicative of the subject's left hemisphere autoregulation state using the left side frequency domain tissue oxygen parameter values and the frequency domain blood pressure values.

In any of the aspects or embodiments described above and herein, the left hemisphere autoregulation data profile may include the left hemisphere COHZ values as a function of the blood pressure level.

In any of the aspects or embodiments described above and herein, the step of evaluating the at least one tissue oxygenation parameter using the second signals and the blood pressure level of the subject using the third signals, relative to one another may include: a) determining right side frequency domain tissue oxygen parameter values by performing a frequency domain transformation of the second signals; b) determining frequency domain blood pressure values by performing a frequency domain transformation of the third signals; and c) determining right hemisphere coherence (COHZ) values indicative of the subject's right hemisphere autoregulation state using the right side frequency domain tissue oxygen parameter values and the frequency domain blood pressure values.

In any of the aspects or embodiments described above and herein, the right hemisphere autoregulation data profile may include the right hemisphere COHZ values as a function of the blood pressure level.

According to another aspect of the present disclosure useful for understanding the presently claimed invention, an apparatus for providing information regarding a subject's cerebral autoregulation function state is provided. The apparatus includes a near infra-red spectroscopy (NIRS) tissue oximeter, a blood pressure sensing device, and a controller. The NIRS tissue oximeter is configured to continuously sense a plurality of tissue regions of the subject. The blood pressure sensing device is configured to continuously measure a blood pressure level of the subject. The controller is in communication with the NIRS tissue oximeter and the blood pressure sensing device. The controller includes at least one processor and a memory device configured to store instructions. The stored instructions when executed cause the controller to: a) control the tissue oximeter to continuously sense at least a portion of a left hemisphere portion of a subject's brain with a tissue oximeter during a period of time, the sensing producing first signals representative of at least one tissue oxygenation parameter within the left hemisphere; b) control the tissue oximeter to continuously sense at least a portion of a right hemisphere portion of a subject's brain with the tissue oximeter during the period of time, the sensing producing second signals representative of the least one tissue oxygenation parameter within the right hemisphere; c) control the blood pressure sensing device to continuously measure a blood pressure level of the subject during the period of time, the measuring producing third signals representative of the blood pressure level of the subject; d) produce a left hemisphere autoregulation data profile using the first signals and the third signals; e) produce a right hemisphere autoregulation data profile using the second signals and the third signals; and f) produce a combined sides autoregulation profile using the left hemisphere autoregulation data profile and the right hemisphere autoregulation data profile.

According to another aspect of the present disclosure useful for understanding the presently claimed invention, a method for providing information regarding a subject's autoregulation function state is provided. The method includes: a) continuously sensing a tissue region of a subject with a tissue oximeter during a period of time, the period of time starting at start time T1, the sensing producing first signals representative of at least one tissue oxygenation parameter; b) continuously measuring a blood pressure level of the subject during the period of time using a blood pressure sensing device, the measuring producing second signals representative of the blood pressure level of the subject; c) evaluating the at least one tissue oxygenation parameter using the first signals and the blood pressure level of the subject using the second signals, relative to one another; d) producing a first start-up profile of autoregulation data representative of the evaluated at least one tissue oxygenation parameter and the blood pressure level relative to one another using the first and said second signals from a portion of the period of time starting at the start time T1 and extending a profile period of time to time T2, wherein the profile period of time is equal to or less than two minutes.

In any of the aspects or embodiments described above and herein, the method may further include replacing the first start-up profile of autoregulation data with a second profile of autoregulation data representative of the evaluated at least one tissue oxygenation parameter and the blood pressure level relative to one another using the first and second signals from a portion of the period of time starting at the time T2 and extending the profile period of time to time T3.

According to another aspect of the present disclosure useful for understanding the presently claimed invention, a method for providing cerebral autoregulation index (CAI) information is provided. The method includes: a) continuously sensing at least a portion of a portion of a subject's brain with a tissue oximeter during a period of time, the sensing producing first signals representative of at least one tissue oxygenation parameter; b) continuously measuring a blood pressure level of the subject during the period of time using a blood pressure sensing device, the measuring producing second signals representative of the blood pressure level of the subject; c) determining values indicative of the subject's autoregulation state using the first signals and the second signals; and d) producing CAI values as a function of time using the values indicative of the subject's autoregulation state.

In any of the aspects or embodiments described above and herein, the method may further include: a) determining frequency domain tissue oxygen parameter values by performing a first frequency domain transformation of the first signals; and b) determining frequency domain blood pressure values by performing a second frequency domain transformation of the second signals. The determined values indicative of the subject's autoregulation state using the first signals and the second signals may be coherence values (COHZ) indicative of the subject's autoregulation state, the COHZ values determined using the frequency domain tissue oxygen parameter values and the frequency domain blood pressure values.

In any of the aspects or embodiments described above and herein, the method may further include displaying the CAI values as a function of time.

In any of the aspects or embodiments described above and herein, the step of producing CAI values as a function of time may include a weight-averaging step wherein at least some of the determined COHZ values are used to produce weight-averaged CAI values.

In any of the aspects or embodiments described above and herein, the method may further include producing an autoregulation profile using the determined COHZ values as a function of the frequency domain blood pressure values. The autoregulation profile may include COHZ values as a function of frequency domain blood pressure bins. The method may further include determining a blood pressure value of the subject and identifying one of bins aligned with the determined blood pressure value. The step of producing CAI values as a function of time may include producing weight-averaged CAI values using the COHZ value associated with the identified bin aligned with the determined blood pressure value, and the COHZ values associated with bins adjacent to the identified bin aligned with the determined blood pressure value.

According to another aspect of the present disclosure useful for understanding the presently claimed invention, an apparatus for providing cerebral autoregulation index (CAI) information is provided. The apparatus includes a near infra-red spectroscopy (NIRS) tissue oximeter, a blood pressure sensing device, and a controller. The NIRS tissue oximeter is configured to continuously sense a tissue region of the subject. The blood pressure sensing device is configured to continuously measure a blood pressure level of the subject. The controller is in communication with the NIRS tissue oximeter and the blood pressure sensing device. The controller includes at least one processor and a memory device configured to store instructions. The stored instructions when executed cause the controller to: a) control the tissue oximeter to continuously sense the tissue region of the subject with the tissue oximeter during a period of time, the sensing producing first signals representative of at least one tissue oxygenation parameter; b) control the blood pressure sensing device to continuously measure a blood pressure level of the subject during the period of time, the measuring producing second signals representative of the blood pressure level of the subject; c) determine values indicative of the subject's autoregulation state using the first signals and the second signals; and d) produce CAI values as a function of time using the values indicative of the subject's autoregulation state.

In any of the aspects or embodiments described above and herein, the stored instructions when executed may cause the controller to further: a) determine frequency domain tissue oxygen parameter values by performing a first frequency domain transformation of the first signals; and b) determine frequency domain blood pressure values by performing a second frequency domain transformation of the second signals. The determined values indicative of the subject's autoregulation state using the first signals and the second signals are coherence values (COHZ) indicative of the subject's autoregulation state, wherein the COHZ values are determined using the frequency domain tissue oxygen parameter values and the frequency domain blood pressure values.

In any of the aspects or embodiments described above and herein, wherein the stored instructions when executed may cause the controller to further to produce weight-averaged CAI values.

According to an aspect of the present disclosure useful for understanding the presently claimed invention, one or more non-transitory computer-readable mediums may be provided comprising instructions for implementing one or more of the present disclosure embodiments described herein.

The foregoing has outlined several aspects of the present invention in order that the detailed description of the invention that follows may be better understood. Additional features and advantages of the invention will be described hereinafter which form the subject of the claims of the invention.

BRIEF DESCRIPTION OF THE DRAWINGS

FIG. 1 is a diagrammatic illustration of autoregulation parameters as a function of time.
FIG. 2 is a diagrammatic illustration of blood flow versus perfusion pressure, indicating a relationship between dilated and constricted blood vessels and autoregulation function.

FIG. 3 is a diagrammatic illustration of cerebral blood flow versus cerebral perfusion pressure, indicating a normal condition, a hypotensive condition, and a hypertensive condition.

FIG. 4A is a diagrammatic representation of an autoregulation system according to an embodiment of the present disclosure.

FIG. 4B is a diagrammatic representation of an autoregulation system according to an embodiment of the present disclosure.

FIG. 5 is a diagrammatic representation of an exemplary ffrequency domain method.

FIG. 6 is an autoregulation profile plot embodiment example.

FIG. 7 is an autoregulation profile plot embodiment example.

FIG. 8 is an autoregulation profile plot embodiment example.

FIG. 9 is a functional diagram of an embodiment of an aspect of the present disclosure.

FIG. 9A is a functional diagram of an embodiment of an aspect of the present disclosure.

FIG. 10 is a diagram of multiple frequency bands shown in FIG. 9 depicted on a frequency axis.

FIG. 10A is a diagram of multiple frequency bands shown in FIG. 9A depicted on a frequency axis.

FIG. 11 is a diagram of multiple frequency bands shown in FIG. 9 depicted on a time axis.

FIG. 11A is a diagram of multiple frequency bands shown in FIG. 9A depicted on a time axis.

FIG. 12 is a diagrammatic display illustrating autoregulation plots for a plurality of NIRS indices.

FIG. 12A provides a diagrammatic example of the present disclosure embodiments that utilize a plurality of different NIRS indices.

FIG. 13 is a chart illustrating an exemplary relationship between phase and frequency.

FIG. 14 is a diagrammatic representation of an embodiment of an exemplary ffrequency domain method according to the present disclosure.

FIG. 15 is an exemplary display of autoregulation data according to an embodiment of the present disclosure.

FIG. 16 is a diagrammatic representation of autoregulation function data, including a historical autoregulation function profile display, a recent autoregulation function profile display, and a combined historical and recent autoregulation function profile display.

FIG. 17 is a diagrammatic representation of autoregulation function data, including a cerebral left side autoregulation function profile display, a cerebral right side autoregulation function profile display, and a combined cerebral autoregulation function profile display.

FIG. 18 is a diagrammatic flow chart of a startup autoregulation function profile methodology.

FIG. 19 is a diagrammatic representation of cerebral autoregulation index (CAI) methodology.

FIG. 20 illustrates a weight averaging technique example for CAI determination.

DETAILED DESCRIPTION

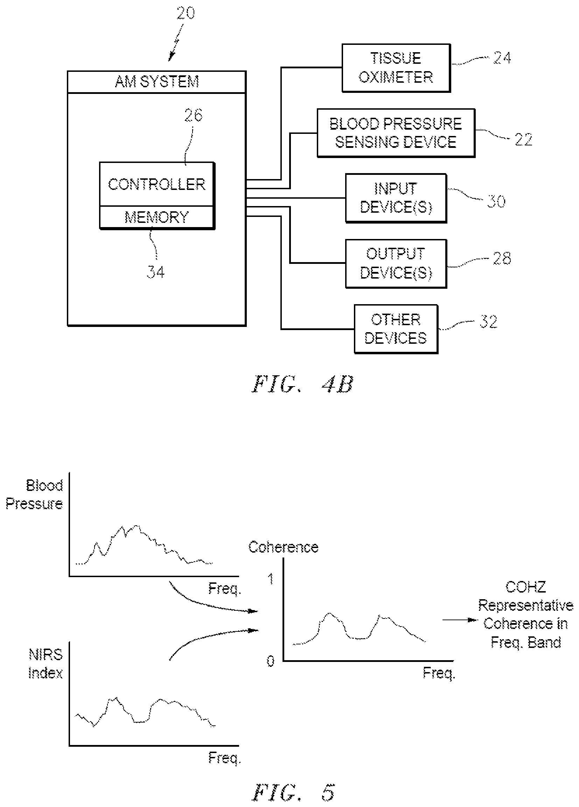

Referring to FIGS. 4A and 4B, a non-limiting embodiment of an autoregulation measurement and monitoring system ("AM system 20") is diagrammatically shown. As will be described herein, the AM system 20 may be configured to produce a data value (e.g., a coherence value) that can be measured and/or monitored, or a data value that is indicative of the state of a subject's autoregulation system function; e.g., the degree to which the subject's autoregulation system is functioning. While an exemplary AM system 20 is shown, the exemplary components illustrated in FIGS. 4A and 4B are not intended to be limiting; e.g., additional or alternative components and/or implementations may be used. In some embodiments (e.g., FIG. 4A), the AM system 20 may include a blood pressure sensing device 22, a tissue oximeter 24, other devices 32, a controller 26, one or more output devices 28, and one or more input devices 30 that may be integrated into a single system device; e.g., a controller 26 integrally connected with sensing hardware (e.g., hardware associated with a tissue oximeter, hardware associated with a blood pressure sensor, etc.). In other embodiments (e.g., FIG. 4B), the AM system 20 may include a controller 26, and may be configured to communicate with (e.g., receive signal data from and/or send signal data to) a blood pressure sensing device 22, a tissue oximeter 24, one or more input devices 30, and one or more output devices 28. In other words, in these embodiments the AM system 20 may be configured to communicate with a blood pressure sensing device 22 that is capable of functioning independently of the AM system 20, a tissue oximeter 24 that is capable of functioning independently of the AM system 20, etc. In other embodiments, the AM system 20 may include some combination of these devices in integral and independent form.

The blood pressure sensing device 22 ("BP sensing device 22") may be any sensor or device configured to continuously determine a subject's blood pressure (e.g., arterial blood pressure). For example, the BP sensing device 22 may a device that is configured to provide continuous blood pressure measurement, such as an arterial catheter line, or a continuous non-invasive blood pressure device, or a pulse oximetry sensor. The present disclosure is not, however, limited to using these particular examples of blood pressure sensing/measuring/monitoring devices 22. The BP sensing device 22 is configured to produce blood pressure value signals indicative of the subject's blood pressure (e.g., arterial blood pressure) during a period of time. The BP sensing device 22 is configured for communication with the AM system controller 26; e.g., send blood pressure value signals to the AM system controller 26, and may receive control signals, etc. from the AM system controller 26. Communications between the BP sensing device 22 and the AM system controller 26 may be by any known means; e.g., hardwire, wireless, etc. The term "continuously" as used herein (to describe a BP sensing device 22 continuously determining a subject's blood pressure) means that the BP sensing device 22 senses and collects subject data on a periodic basis during the monitoring time period, which periodic basis is sufficiently frequent that it may be considered to be clinically continuous. For example, some BP sensing devices 22 sample data every ten seconds or less and can be configured to sample data more frequently (e.g., every two seconds or less).

The tissue oximeter 24 may be a device configured to continuously sense a tissue oxygenation parameter (referred to hereinafter individually as a "NIRS index" or collectively as "NIRS indices") that varies with blood flow in a subject's tissue; e.g., tissue oxygen saturation ($StO_2$), total hemoglobin blood volume (THb), relative total hemoglobin blood volume (rTHb), differential changes in oxyhemoglobin ($HbO_2$) and deoxyhemoglobin (Hb), HbD (i.e., $HbO_2$ - Hb), etc. An example of an acceptable tissue oximeter 24 is a near infra-red spectroscopy ("NIRS") type tissue oximeter ("NIRS tissue oximeter"). U.S. Patent Nos. 6,456,862; 7,072,701; 8,078,250; 8,396,526; and 8,965,472; and 10,117,610 disclose non-invasive NIRS tissue oximeter that may be used within the present disclosure. The term "continuously" as used herein (to describe a tissue oximeter 24 continuously sensing a tissue oxygenation parameter) means that the tissue oximeter 24 senses and collects subject data on a periodic basis during the monitoring time period, which periodic basis is sufficiently frequent that it may be considered to be clinically continuous. For example, some tissue oximeters 24 sample data every ten seconds or less and can be configured to sample data more frequently (e.g., every two seconds or less).

The tissue oximeter 24 includes one or more sensors in communication with a controller portion. Each sensor includes one or more light sources (e.g., light emitting diodes, or "LEDs") and one or more light detectors (e.g., photodiodes, etc.). The light sources are configured to emit light at different wavelengths of light, e.g., wavelengths of light in the red or near infrared range; 400-1000nm. In some sensor embodiments, a sensor may be configured to include a light source, a near detector(s), and a far detector(s). The near detector(s) are disposed closer to the light source than the far detector(s). A non-limiting example of such a sensor is disclosed in U.S. Patent No. 8,965,472. The tissue oximeter 24 is configured for communication with the AM system controller 26; e.g., send signals representative of one or more NIRS indices to the AM system controller 26, and may receive control signals, etc. from the AM system controller 26. Communications between the tissue oximeter 24 and the AM system controller 26 may be by any known means; e.g., hardwire, wireless, etc.

The NIRS tissue oximeter 24 utilizes one or more algorithms for determining one or more of the NIRS indices. The present disclosure is not limited to any particular NIRS tissue oximeter 24 or any algorithm for determining a NIRS Index of the sensed tissue. U.S. Patent Nos. 9,913,601; 9,848,808; 9,456,773; 9,364,175; 9,923,943; 8,788,004; 8,396,526; 8,078,250; 7,072,701; and 6,456,862 all describe non-limiting examples of algorithms for determining NIRS indices that may be used with the present disclosure.

One or both of the BP sensing device 22 or the tissue oximeter 24 may be further configured to measure other parameters, such as respiratory rate, respiratory effort, heart rate, etc. The BP sensing device 22 and the tissue oximeter 24 may be placed on the same or different parts of the patient's body.

As stated above, the BP sensing device 22, the tissue oximeter 24, and other devices 32 identified herein may be integrated within the AM system 20, or they may be independent devices that provide signal data to the AM system 20, or any combination thereof. In those embodiments wherein one or more of the aforesaid devices is independent of the AM system 20, that independent device may be in communication with the AM system controller 26 in any manner.

As stated above, the AM system 20 includes a controller 26, and may include one or more output devices 28 and one or more input devices 30. Non-limiting examples of an input device 30 include a keyboard, a touchpad, or other device wherein a user may input data, commands, or signal information, or a port configured for communication with an external input device via hardwire or wireless connection, etc. Non-limiting examples of an output device 28 include any type of display, printer, or other device configured to display or communicate information or data produced by the AM system 20. The AM system 20 may be configured for connection with an input device 30 or an output device 28 via a hardwire connection or a wireless connection.

In some embodiments, the AM system controller 26 may be configured (e.g., via electrical circuitry) to process various received signals (received from integral or independent devices) and may be configured to produce certain signals to the same; e.g., signals configured to control one or more components within the AM system 20. Alternatively, the AM system 20 may be configured such that signals from the respective component are sent to one or more intermediate processing devices, and the intermediate processing device may in turn provide processed signals or data to the AM system controller 26. As will be explained below, the AM system controller 26 may be configured to execute stored instructions (e.g., algorithmic instructions) that cause the AM system 20 to perform steps or functions described herein, to produce data (e.g., measurements, etc.) relating to a subject's autoregulation system, to communicate, etc.

The AM system controller 26 may include any type of computing device, computational circuit, or any type of process or processing circuit capable of executing a series of instructions that are stored in memory 34. The controller 26 may include multiple processors and/or multicore CPUs and may include any type of processor, such as a microprocessor, digital signal processor, co-processors, a micro-controller, a microcomputer, a central processing unit, a field programmable gate array, a programmable logic device, a state machine, logic circuitry, analog circuitry, digital circuitry, etc., and any combination thereof. For example, in those embodiments of the AM system 20 described above that include multiple components integral with the system 20 (e.g., a blood pressure sensing device 22, a tissue oximeter 24, etc.) integral with the system, the controller 26 may include multiple processors; e.g., an independent processor dedicated to each respective component, any and all of which processors may be in communication with a central processor of the AM system 20 that coordinates functionality of the controller 26 / AM system 20. The instructions stored in memory may represent one or more algorithms for controlling the AM system 20, and the stored instructions are not limited to any particular form (e.g., program files, system data, buffers, drivers, utilities, system programs, etc.) provided they can be executed by the controller 26. The instructions are configured to perform the methods and functions described herein.

The memory 34 may be a non-transitory machine readable storage medium configured to store instructions that when executed by one or more processors, cause the one or more processors to perform or cause the performance of certain functions. The memory 34 may be a single memory device or a plurality of memory devices. A memory device may include a storage area network, network attached storage, as well as a disk drive, a read-only memory, random access memory, volatile memory, non-volatile memory, static memory, dynamic memory, flash memory, cache memory, and/or any device that stores digital information. One skilled in the art will appreciate, based on a review of this disclosure, that the implementation of the controller 26 may be achieved via the use of hardware, software, firmware, or any combination thereof.

Implementation of the techniques, blocks, steps, and means described herein may be done in various ways. For example, these techniques, blocks, steps and means may be implemented in hardware, software, or a combination thereof. For a hardware implementation, processing devices configured to carry out the described functions and steps (e.g., by executing stored instructions) may be implemented within one or more application specific integrated circuits (ASICs), digital signal processors (DSPs), digital signal processing devices (DSPDs), programmable logic devices (PLDs), field programmable gate arrays (FPGAs), processors, controllers, micro-controllers, microprocessors, other electronic units designed to perform the functions described herein and/or a combination thereof.

Also, it is noted that the embodiments of the present disclosure may be described herein as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The present AM system 20 utilizes real-time data collection of tissue oximeter 24 data (e.g., relating to one or more NIRS indices) and continuous blood pressure measurement data to produce data relating to a subject's autoregulation function. The specific functionality of the tissue oximeter 24 and the BP sensing device 22 (e.g., sampling rate, etc.) can be set as appropriate for the operation of the AR system 20, and the present disclosure is not limited to any particular device settings. The tissue oximeter 24 data and the BP sensing device 22 data (e.g., in signal form) are sent to the AR system controller 26 where they are processed using stored instructions to determine autoregulation function data. The autoregulation function data reflects the relationship between at least one NIRS index and the blood pressure of a subject. As described herein, changes in a NIRS index and changes in blood pressure level relative to one another can be a product of the subject's autoregulation system function. The present disclosure is configured to evaluate those changes. The present disclosure describes how coherence values ("COHZ") may be determined as an indicator of the relationship between a NIRS index and blood pressure. The present disclosure is not, however, limited to autoregulation data in the form of COHZ values. For example, the present AM system 20 may be configured to produce autoregulation function data indicative of a correlation (e.g., based on a time domain) between at least one NIRS Index and blood pressure data to determine autoregulation data for a subject.

FIG. 5 diagrammatically depicts an exemplary frequency domain method that involves taking synchronous blood pressure and NIRS index values over a predetermined sampling window (e.g., period of time). As stated herein, aspects of the present disclosure are not limited to using a frequency domain method. In this exemplary frequency domain method, the blood pressure and NIRS index values are each transformed (e.g., via a Fourier transformation) from a time domain to a frequency domain (shown as respective plots of blood pressure versus frequency and NIRS Index versus frequency; the transformed tissue oxygenation parameter values (e.g., the NIRS index) may be referred to as a "frequency domain tissue oxygenation parameter values", and the transformed blood pressure values may be referred to as "frequency domain blood pressure values"), and the transformed data is further analyzed to determine the degree of coherence there between within a single band of frequencies (i.e., a single frequency band). The degree of coherence may be indicated in terms of an arbitrarily assigned scale of zero to one (0 - 1), wherein the degree of coherence increases from zero to one (shown as a plot of coherence values versus frequency). A coherence value of one represents a pressure passive condition as described above. Conversely, a coherence value that approaches zero indicates increasingly less relationship between relation between the NIRS index and blood pressure parameters. A coherence value ("COHZ") that is representative of substantially all frequencies in the single frequency band may be used as an autoregulation index ("AR Index") or pressure passive index ("PPI"). For purposes of the present description, the terms "AR Index" and pressure passive index "PPI" are intended to be substantially equivalent, and for sake of clarity the term "AR Index" will be used hereinafter. The representative coherence value ("COHZ") for a single frequency band may be an average of the coherence values within the single frequency band, or a mean value, or a median value, or any similar value that collectively represents the coherence values over all frequencies in the single frequency band.

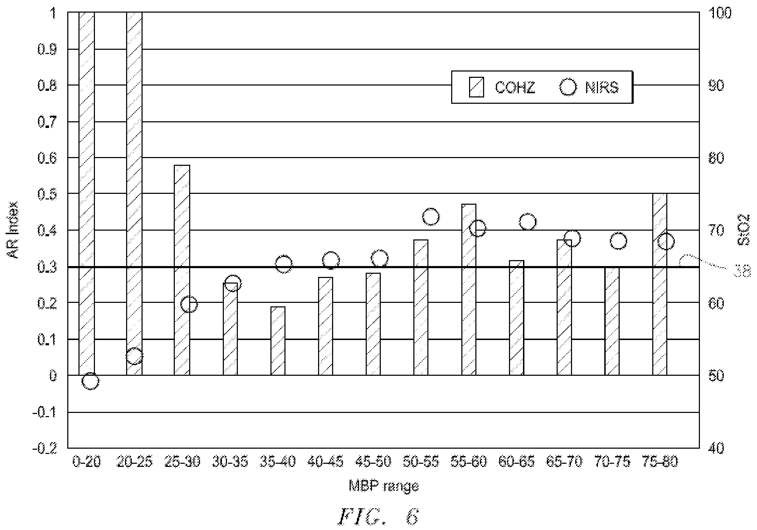

In some embodiments, the COHZ values (within the single frequency band) determined over a period of time may be binned in blood pressure increments (e.g., every 5 mmHg) or in incremental blood pressure ranges (e.g., 0-20 mmHg, 20-25 mmHg, 25-30 mmHg, etc.). Non-limiting examples of autoregulation profile plots over a few hours are shown in FIGS. 6-8, which autoregulation profile plots are based on COHZ values determined within a single frequency band.

In FIG. 6, an autoregulation profile plot based on pig lab data is shown, depicting Y-axes of an AR Index and a representative $StO_2$ value (i.e., a NIRS index), an X-axis of a representative blood pressure range (shown in 5 mmHg bins), and coherence values ("COHZ") per blood pressure bin. The representative $StO_2$ value may be a mean value, an average value, a median value, or similar value that collectively represents the $StO_2$ values over all frequencies in the band. In alternative embodiments, the autoregulation profile plot may include a NIRS index other than $StO_2$; i.e., THb, rTHb, differential changes in $HbO_2$ and Hb, HbD, etc. As can be seen in FIG. 6, the COHZ values may be viewed in terms of the AR Index. The data depicted in FIG. 6 indicates that the autoregulation of the subject pig becomes increasingly pressure passive at a blood pressure value less than about thirty mmHg (30 mmHg). FIG. 6 includes a horizontal line 38 at about the AR Index value of 0.3 to reflect an AR Index value inflection point above which the subject's autoregulation system may be described as being pressure passive to some degree (e.g., the degree to which the subject's autoregulation system is pressure passive increases as the AR Index approaches an AR Index value of 1), and below which the subject's autoregulation function is substantially normal. The present disclosure is not limited to the AR Index value inflection point of 0.3, or to any particular AR Index value inflection point. The AR Index value inflection point may be based on empirical data, and may vary depending on factors such as characteristics of the subject; e.g., age, health, smoker, etc.

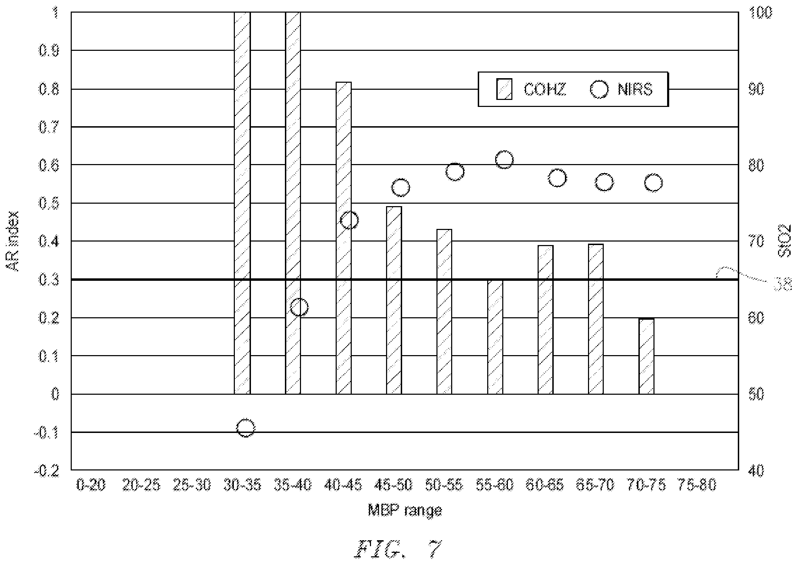

In FIG. 7, an autoregulation profile plot based on human neonate data is shown, depicting Y-axes of an AR Index and a representative $StO_2$ (i.e., a NIRS Index), an X-axis of a representative blood pressure range (shown in 5 mmHg bins), and coherence values ("COHZ") per blood pressure bin. As stated above, the autoregulation profile plot may include a NIRS Index other than $StO_2$; i.e., THb, differential changes in $HbO_2$ and Hb, HbD, etc. The data depicted in FIG. 7 indicates that the autoregulation of the human neonate subject becomes increasingly pressure passive at a blood pressure value less than about fifty mmHg (50 mmHg). FIG. 7 includes a horizontal line 38 at about the AR Index value of 0.3 to reflect an AR Index value inflection point above which the subject's autoregulation system may be described as being pressure passive to some degree, and below which the subject's autoregulation function is substantially normal. As stated above, the present disclosure is not limited to the AR Index value inflection point of 0.3, or to any particular AR Index value inflection point.

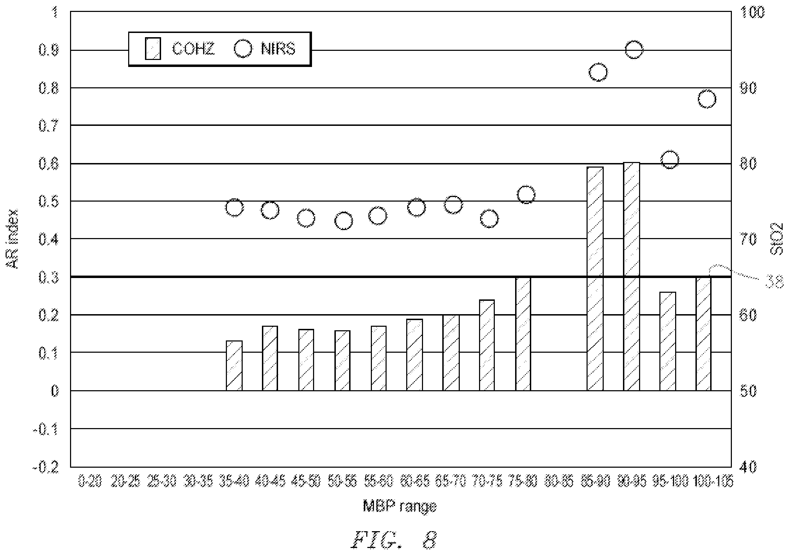

In FIG. 8, an autoregulation profile plot based on human neonate data is shown, depicting Y-axes of an AR Index and a representative $StO_2$ (i.e., a NIRS Index), an X-axis of a representative blood pressure range (shown in 5 mmHg bins), and coherence values ("COHZ") per blood pressure bin. As stated above, the autoregulation profile plot may include a NIRS index other than $StO_2$; i.e., THb, rTHb, differential changes in $HbO_2$ and Hb, HbD, etc. The data depicted in FIG. 8 indicates that the autoregulation of the human neonate subject becomes increasingly pressure passive at a blood pressure value greater than about eighty-five mmHg (85 mmHg). FIG. 8 includes a horizontal line 38 at about the AR Index value of 0.3 to reflect an AR Index value inflection point above which the subject's autoregulation system may be described as being pressure passive to some degree, and below which the subject's autoregulation function is substantially normal. As stated above, the present disclosure is not limited to the AR Index value inflection point of 0.3, or to any particular AR Index value inflection point.

Aspects of the present disclosure may provide enhanced measurement of a subject's autoregulation function (e.g., the degree to which a subject's autoregulation system is functioning), or an enhanced determination of the state of the subject's autoregulation function. For example, in some embodiments the present disclosure includes determining and analyzing COHZ values from different predetermined frequency bands simultaneously (or nearly simultaneously) from NIRS tissue oximetry and physiological (e.g., mean blood pressure) data taken from different sampling windows, and determining a peak COHZ value (i.e., a "MAX COHZ" value) at a given point in time from the COHZ values determined within the different predetermined frequency bands. The MAX COHZ value may be determined periodically (e.g., every 30 seconds). In this way, the MAX COHZ value used for further analysis could be based on the COHZ value determined from any of the different predetermined frequency bands; e.g., at a first point in time the MAX COHZ value may be based on data from a first frequency band, and at another point in time the MAX COHZ value may be based on data from a different frequency band, etc. As will be explained below, the possibility of determining a MAX COHZ value from a plurality of different predetermined frequency bands, as opposed to it being determined from a single frequency band, is believed to increase the sensitivity and accuracy of the AM system 20, and to improve the real-time response detection of the AM system 20 (e.g., improve the ability of the AM system 20 to more rapidly detect an issue with a subject's autoregulation function).

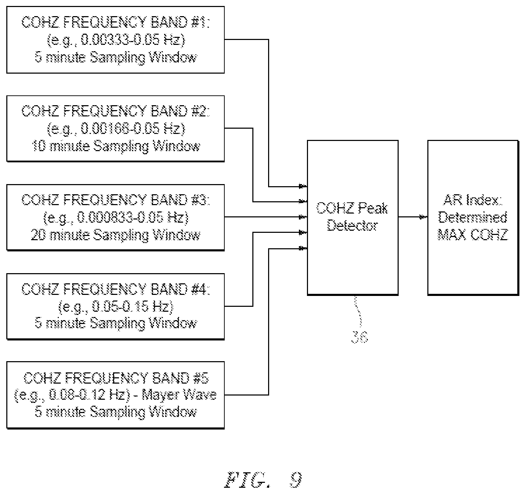

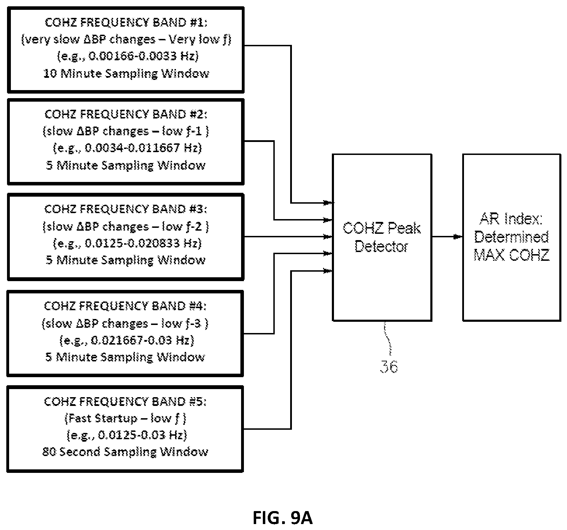

Referring to FIG. 9, a representative coherence value ("COHZ") may be determined in at least a plurality of the predetermined frequency bands (e.g., in a method similar to that described above with respect to FIG. 5), and a real-time peak coherence value (MAX COHZ) may be determined from those COHZ values (i.e., determined from the COHZ values determined in a respective different frequency band). For example, in the exemplary methodology shown in FIG. 9, there are five different frequency bands shown. Frequency band number 1 ("#1") has a bandwidth of 0.00333 Hz to 0.05 Hz and a five minute sampling window. Frequency band number 2 ("#2") has a bandwidth of 0.00166 Hz to 0.05 Hz and a ten minute sampling window. Frequency band number 3 ("#3") has a bandwidth of 0.000833 Hz to 0.05 Hz and a twenty minute sampling window. Hence, frequency band numbers #1-3 represent different bandwidths and different sampling windows; e.g., the range of frequencies within frequency band numbers #1-3 are chosen at least in part based on the duration of the associated sampling window; e.g., 5 mins, 10 mins, 20 mins, etc. The range of frequencies within a frequency band may also be chosen in view of the sampling rate of the tissue oximeter 24, or the sampling rate (or collection rate) of the blood pressure sensing device 22, or both or some combination thereof; e.g., the frequency band may be chosen such that the sampling rate of the respective device is within the frequency band. Frequency band #1 is understood to be effective for identifying coherence (e.g., a coherence that is readily identifiable) when there is a rapid change in a subject's blood pressure. Frequency band #2 is understood to be effective for identifying coherence (e.g., a coherence that is readily identifiable) when changes in a subject's blood pressure are less rapid than those considered within frequency band #1. Frequency band #3 is understood to be effective for identifying coherence (e.g., a coherence that is readily identifiable) when changes in a subject's blood pressure are less rapid than those considered within frequency band #2. The frequency ranges for frequency bands #1-3 described above are examples, and the present disclosure is not limited to these particular frequency ranges. Frequency band number #4 has a bandwidth of 0.05 Hz to 0.15 Hz and a five minute sampling window. The range of frequencies within frequency band #4 is chosen to permit evaluation of a range of frequencies higher than those within frequency band #1-3 and is understood to be effective for identifying coherence (e.g., a coherence that is readily identifiable) when there is a rapid change in a subject's blood pressure, and/or may be chosen to reflect respiratory effects (e.g., breathing rate, etc.). The frequency range for frequency band #4 described above is also an example, and the present disclosure is not limited to this particular frequency range. Frequency band number #5 has a bandwidth of 0.08 Hz to 0.12 Hz and a five minute sampling window. The range of frequencies within frequency band #5 may be chosen to evaluate physiological characteristics of the subject (e.g., Mayer waves), and is understood to be effective for identifying coherence (e.g., a coherence that is readily identifiable) associated with Mayer waves. Mayer waves are cyclic changes (e.g., "waves") in arterial blood pressure brought about by oscillations in baroreceptor and chemoreceptor reflex control systems. Mayer waves may be defined as arterial blood pressure oscillations at frequencies slower than respiratory frequency and which show the strongest, significant coherence (strength of linear coupling between fluctuations of two variables in the frequency domain) with efferent sympathetic nervous activity. The frequency range for frequency band #5 is also an example, and the present disclosure is not limited to this particular frequency range.

FIG. 9A illustrates an embodiment like that described above wherein COHZ values may be determined in a plurality of the predetermined frequency bands (different bands than those identified in FIG. 9), and a real-time MAX COHZ value may be determined from those COHZ values. In this embodiment, frequency band number 1 ("#1") has a bandwidth of 0.00166 Hz to 0.0033 Hz and a ten minute sampling window. Frequency band number 2 ("#2") has a bandwidth of 0.0034 Hz to 0.011667 Hz and a five minute sampling window. Frequency band number 3 ("#3") has a bandwidth of 0.0125 Hz to 0.020833 Hz and a five minute sampling window. Frequency band number 4 ("#4") has a bandwidth of 0.021667 Hz to 0.03 Hz and a five minute sampling window. Frequency band number 5 ("#5") has a bandwidth of 0.0125 Hz to 0.03 Hz and an eighty second (80 secs.) sampling window. As indicated in FIG. 9A, frequency band number 1 is useful for identifying coherence involving very slow changes in blood pressure, frequency band numbers 2-4 are useful in identifying coherence relating to different degrees of slow changes in blood pressure, and frequency band number 5 is useful for identifying coherence during a fast startup period as will be described herein. More specifically, the relatively short sampling window of frequency band number 5 can be used to give an initial and quicker assessment of cerebral autoregulation index (CAI) and autoregulation function at the start of a monitoring period. The longer sampling windows (e.g., 5 and 10 minute windows) need more time to fill the sampling windows to permit a CAI value to be computed at the start of the respective monitoring period; e.g., coherence cannot be computed until the sampling window has sufficient data to perform the necessary processing (e.g., FFTs) that is utilized for coherence computation. After a longer sampling window (e.g., a 5 minute window) has sufficient data to compute CAI data, the use of the "startup" window (e.g., 80 seconds) can be optionally dropped or ignored for CAI computation. The frequency ranges for frequency bands #1-5 described above are examples, and the present disclosure is not limited to these particular frequency ranges. The time duration of the sampling windows for frequency bands #1-5 described above are examples, and the present disclosure is not limited to these particular sampling window durations.

Embodiments of the present disclosure that determine a MAX COHZ from a plurality of predetermined frequency bands are not limited to the above disclosed frequency bands or the identified sampling windows; e.g., fewer, or more bands associated with different duration sampling windows may be used, and/or different sampling windows may be used, etc. The above-disclosed frequency bands and sampling windows are understood to provide considerable utility as will be described below, but the present disclosure is not limited thereto.

By determining COHZ values within a plurality of predefined frequency bands (e.g., like those shown in FIGS. 9 and 9A), the highest COHZ value (i.e., the MAX COHZ value) can be selected from the different frequency bands via a COHZ peak detector 36 at any given point in time (e.g., including periodic determinations as indicated above). The MAX COHZ value provides greater sensitivity to autoregulation function at any given point in time as compared to a COHZ value determined from a single frequency band; e.g., as shown in methodology depicted in FIG. 5. As a result, the MAX COHZ value (and corresponding AR Index) is more indicative of the real-time (present time) circumstances and the clinician can be alerted more rapidly especially if the subject's blood pressure falls below the lower autoregulation threshold (e.g., the "LLA", which in a graph solution appears as a lower blood pressure deflection point). For example, if there is a rapid change in a subject's blood pressure and in a NIRS index (e.g., $StO_2$), the COHZ value determined from a higher frequency band will likely be substantially higher than the COHZ value determined from a lower frequency band. Hence, the "event" (i.e., the rapid change in a subject's blood pressure and in a NIRS index) is more rapidly identified within the higher frequency band. Conversely if there is a slow simultaneous change in a subject's blood pressure and in a NIRS index (e.g., $StO_2$), the COHZ value determined from a lower frequency band will likely be substantially higher than the COHZ value determined from a higher frequency band. Hence, the "event" (i.e., the slow change in a subject's blood pressure and in a NIRS index) is more rapidly identified within the lower frequency band.

There is significant clinical value in determining an indication of change in a subject's autoregulation functioning (e.g., if the autoregulation function is failing, such as a pressure passive condition, etc.) as quickly as possible. Autoregulation monitoring systems that monitor a subject's autoregulation functioning via a frequency domain approach that utilizes a single frequency band may be slower to report a high coherence value, or the magnitude of a coherence value may be diluted by lower coherence values at lower frequencies due to the averaging of all individual frequency coherence values. Embodiments of the present disclosure mitigate these limitations by determining COHZ values within a plurality of predefined frequency bands and determining a MAX COHZ value therefrom.

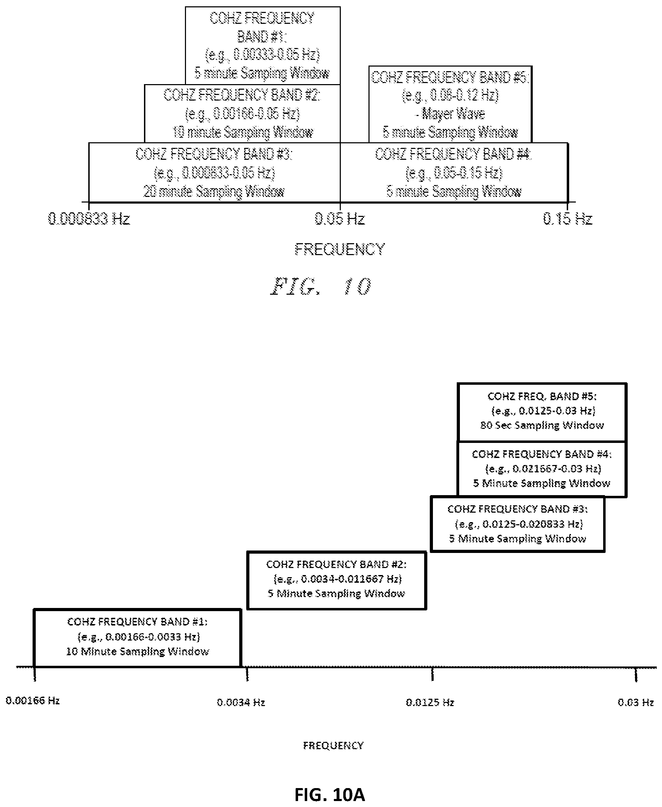

The diagrammatic illustration shown in FIG. 10 depicts a frequency domain methodology such as that shown in FIG. 9 and described above. The diagrammatic illustration shown in FIG. 10A depicts a frequency domain methodology such as that shown in FIG. 9A and described above. In FIGS. 10 and 10A, the predetermined frequency bands #1-5 are shown on a horizontal frequency axis to illustrate the differences in the respective frequency bands.

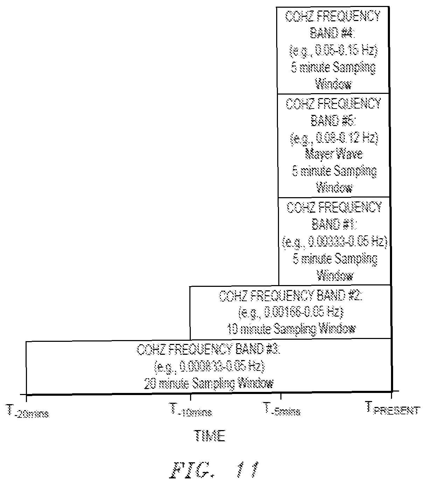

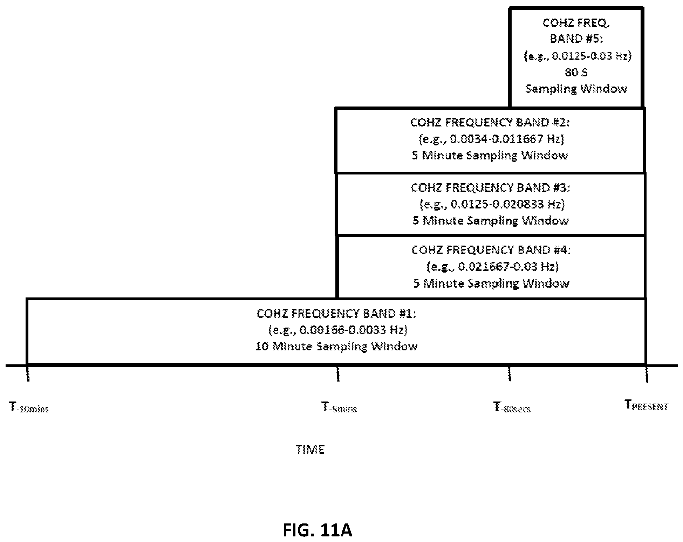

The diagrammatic illustration shown in FIG. 11 shows time domain sampling windows corresponding to the exemplary predetermined frequency bands #1-5 shown in FIG. 9 and described above. The diagrammatic illustration shown in FIG. 11A shows time domain sampling windows corresponding to the exemplary predetermined frequency bands #1-5 shown in FIG. 9A and described above. The orientation of the time domain sampling windows shown in FIG. 11 illustrates that in some embodiments of the present disclosure, the autoregulation data produced at a given point in time ("$T_{Present}$") may be based on the time sampling windows representative of the immediate past 5 minutes ("$T_{-5mins}$"), 10 minutes ("$T_{-10mins}$"), and 20 minutes ("$T_{-20mins}$"); i.e., sampling windows that coincide at least partially. The orientation of the time domain sampling windows shown in FIG. 11A illustrates that in some embodiments of the present disclosure, the autoregulation data produced at a given point in time ("$T_{Present}$") may be based on the time sampling windows representative of the immediate past 80 seconds ("$T_{-80secs}$"), 5 minutes ("$T_{-5mins}$"), and 10 minutes ("$T_{-10mins}$"); i.e., sampling windows that coincide at least partially. As stated above, the relatively short sampling window (e.g., 80 seconds) of frequency band number 5 can be used to give an initial and quicker assessment of CAI and autoregulation function at the start of a monitoring period. The longer sampling windows (e.g., 5 and 10 minute windows) need more time to fill the sampling windows to permit a CAI value to be computed at the start of the respective monitoring period; e.g., coherence cannot be computed until the sampling window has sufficient data to perform the necessary processing (e.g., FFTs) that is utilized for coherence computation. After a longer sampling window (e.g., a 5 minute window) has sufficient data to compute CAI data, the use of the "startup" window (e.g., 80 seconds) can be optionally dropped or ignored for CAI computation. As stated above, the frequency ranges and the time duration of the sampling windows for frequency bands #1-5 described above are nonlimiting examples.

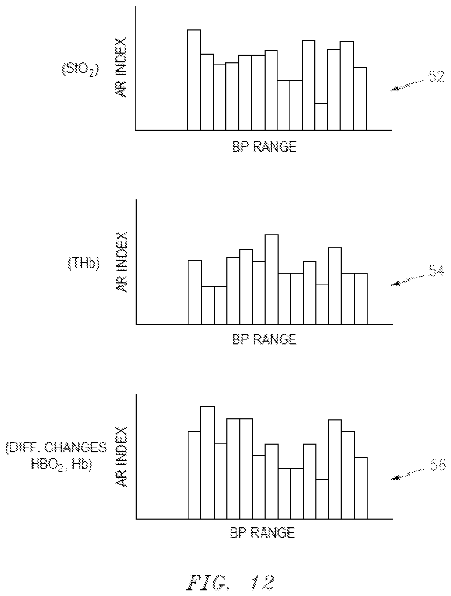

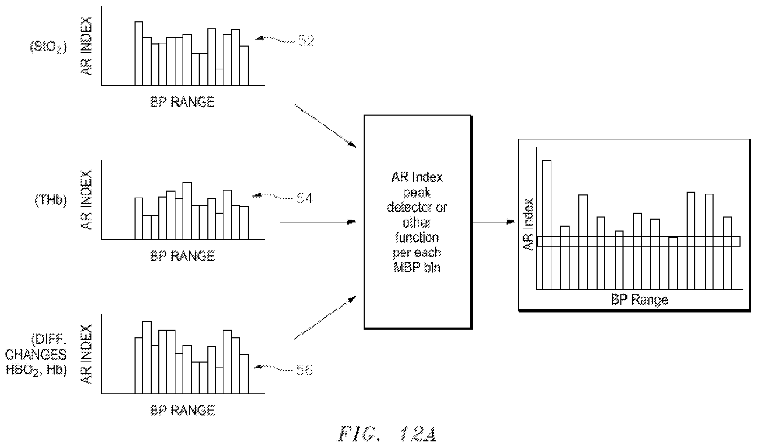

Other aspects of the present disclosure may also provide enhance measurement of a subject's autoregulation function. As described above, a subject's autoregulation functioning may be evaluated using synchronous blood pressure and NIRS index values over a period of time, where the blood pressure and NIRS index values are each transformed from a time domain to a frequency domain, and the transformed data is further analyzed to determine the degree of coherence there between. In some embodiments of the present disclosure, this process may be executed for a plurality of different NIRS indices (e.g., executed using at least two of $StO_2$, THb, rTHb, differential changes in $HbO_2$ and Hb, HbD, etc.). In an instance where one NIRS index is more sensitive to autoregulation function than another, performing the autoregulation function determination processes as described herein (e.g., within a single frequency band, or within a plurality of frequency bands) can provide additional sensitivity and/or faster identification of change in a subject's autoregulation function. FIG. 12, for example, shows a first autoregulation plot 52 (AR Index v. BP Range) based on a first NIRS index (e.g., $StO_2$), a second autoregulation plot 54 based on a second NIRS index (e.g., THb), and a third autoregulation plot 56 based on a third NIRS index (e.g., differential changes in $HbO_2$ and Hb). The COHZ values for each of the aforesaid NIRS indices can be evaluated relative to one another on a per blood pressure bin basis; e.g., the COHZ value associated with $StO_2$ for the 50-55 mmHg bin, the COHZ value associated with THb for the 50-55 mmHg bin, and the COHZ value of the differential changes in $HbO_2$ and Hb for the 50-55 mmHg bin, etc. In some embodiments, the evaluation process may include choosing the NIRS index with the highest COHZ value for that bin. FIG. 12A provides a diagrammatic example of the above methodologies, as well as a diagrammatic view of how the aforesaid methodologies may be displayed. In other embodiments, the evaluation process may include creating an average COHZ value (or a mean or median value, etc.) based on the COHZ values for the aforesaid NIRS indices for that bin. In some instances, a first NIRS index value may be more sensitive to autoregulation function than another (or in other instances, one NIRS index may be affected by a physiological event, whereas another NIRS index is not affected - or is less affected by the same physiological event) and performing the autoregulation function determination processes as described above can provide additional sensitivity and/or faster identification of change in a subject's autoregulation function. The present disclosure is not limited to any particular methodology for monitoring a subject's autoregulation functioning using a plurality of different NIRS indices. For example in a first embodiment, the methodologies described herein for determining a MAX COHZ value can be performed for each NIRS index, and the MAX COHZ values from each such determination (i.e., MAX $COHZ_{NIRS\ INDEX\ 1}$, MAX $COHZ_{NIRS\ INDEX\ 2}$, MAX $COHZ_{NIRS\ INDEX\ 3}$, etc.) may then be evaluated relative to one another to choose a maximum value therefrom (e.g., a MAX $COHZ_{NIRS\ INDICES}$) that may then be used to evaluate the subject's autoregulation function as described herein. As another example, the plurality of different NIRS indices may be utilized elsewhere (e.g., earlier) in the MAX COHZ value determination. For example, during the processes for determining a COHZ value for each frequency band, a COHZ value may be determined for each NIRS Index within a particular frequency band (e.g., for a first frequency band: $COHZ_{NIRS\ INDEX\ 1-FB1}$, $COHZ_{NIRS\ INDEX\ 2-FB1}$, $COHZ_{NIRS\ INDEX\ 3-FB1}$), and a peak COHZ value chosen therefrom, and the process repeated for each frequency band. A peak coherence value (MAX COHZ) may then be determined from the aforesaid COHZ values; e.g., in a manner described herein. These exemplary methodologies for monitoring a subject's autoregulation functioning using a plurality of different NIRS indices are meant to be illustrative and not limiting.

In some embodiments, once a MAX COHZ value is determined from the coherence values (COHZ) determined from a plurality of predetermined frequency ranges being analyzed at that moment of time, the MAX COHZ value may be binned in blood pressure ranges (e.g., every 5 mmHg); e.g., if a small change in blood pressure is detected. In some embodiments, MAX COHZ values may be continuously determined on a periodic basis (e.g., every 30 seconds) over a given period of time (e.g., hours) and those MAX COHZ values may be further processed, for example, to facilitate display of the information. For example, periodically determined MAX COHZ values collected over a period of time may be binned and a representative value of the binned values (e.g., an average, mean, or median value) may be displayed within an autoregulation profile plot; e.g., a plot structured similar to those shown in FIGS. 6-8. In those embodiments that include a binning process wherein a representative value is determined for each bin, the process of producing the representative value (e.g., determining an average, mean, or median value) may provide an additional advantage of mitigating outlier values (e.g., false positives and false negatives).

A NIRS index change or a blood pressure change does not necessarily implicate a subject's autoregulation function. An autoregulation function is typically in response to related changes in a NIRS index and blood pressure. For example, if a NIRS index changes within a relatively short period of time (e.g., 30 seconds) of a blood pressure change, then COHZ values derived from NIRS index changes and blood pressure changes are likely attributable to the subject's physiology and represent a valid indicator of autoregulation function. Conversely, consider a NIRS index change that occurs a relatively long period of time (e.g., 2 minutes) after a blood pressure change. The temporal separation between these two events makes it less likely that they related to one another as a physiological response. Hence, COHZ values derived from these temporally distinct changes are less likely attributable to the subject's physiology and the COHZ values would likely be a poor indicator of autoregulation function. The temporally distinct changes are more likely attributable to other physiological events such as hypoxia or outside interference such as subject movement.

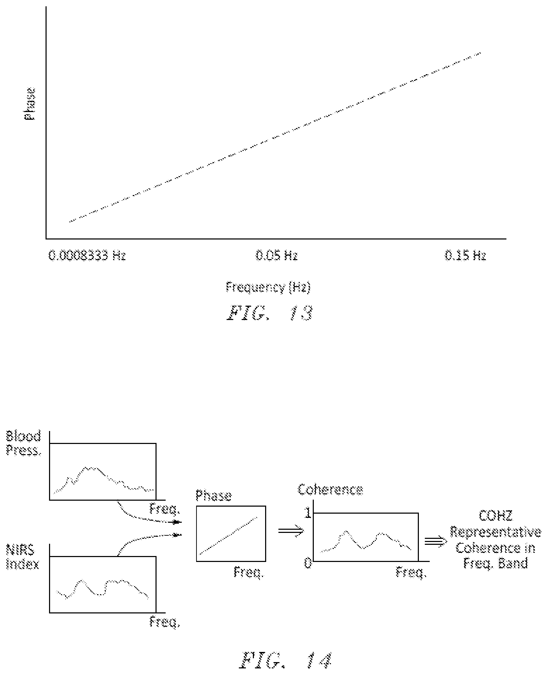

Referring to FIGS. 13 and 14, embodiments of the present disclosure consider a temporal relationship between NIRS index changes and blood pressure changes in evaluating a subject's autoregulation function. For example, in some embodiments, coherence values determined in particular frequency bands may be evaluated in terms of a "phase" range. The terms "phase" or "phase range" as used herein are used to mean a predetermined temporal relationship between the NIRS index change occurrence and the blood pressure change occurrence, or a frequency relationship between the NIRS index change occurrence and the blood pressure change. For example, a phase may be defined as:

$$\frac{Predetermined\ NIRS\ Index\ Response\ time\ to\ a\ change\ in\ Blood\ Pressure}{1/frequency} \qquad [Eqn.\ 2]$$

The above mathematical relationship is a non-limiting example of how the term "phase" may be defined, and the present disclosure is not limited to this particular mathematical relationship. In some embodiments, the phase relationship between the NIRS index change occurrence and the blood pressure change may be expressed in terms of the relationship between the aforesaid values expressed in a frequency domain, and the extent to which the aforesaid values in a frequency domain are out of phase with one another.

To illustrate how phase may be used to evaluate the validity of coherence values, consider coherence values determined within a particular frequency band (e.g., a very low frequency band). If the phase (e.g., the time separation between the change in blood pressure and the change in NIRS index) is outside of a predetermined phase range, then the respective determined coherence value can be discarded, or assigned a value (e.g., a low value such as zero) that will not corrupt the COHZ determination for that particular frequency band. The phase evaluation of an individual frequency may be performed before the coherence values for the particular frequency band are processed (e.g., averaged) to produce the COHZ value for that particular frequency band. As shown in FIG. 13, the maximum phase allowable as a function of NIRS response time to blood pressure change increases with frequency; e.g., at higher frequencies, all phase values may be physiologically valid when evaluating a subject's autoregulation function, whereas at very low frequencies only limited phase values may be physiologically valid (e.g., the temporal relationship between the blood pressure change and the NIRS Index change is too great and therefore less likely attributable to the subject's physiology) when evaluating a subject's autoregulation function.

In some instances, a subject may experience an acute blood pressure drop that may go below or above a lower autoregulation blood pressure range. In such instances, the present AR system may be configured (e.g., via stored algorithmic instructions) to update the displayed autoregulation information, including an autoregulation profile plot. The displayed information may include high values above a predetermined AR Index (or PPI Index) value indicative of a threshold autoregulation function (which value may be depicted as an AR Index value inflection line) above which the subject's autoregulation function becomes increasingly pressure passive.

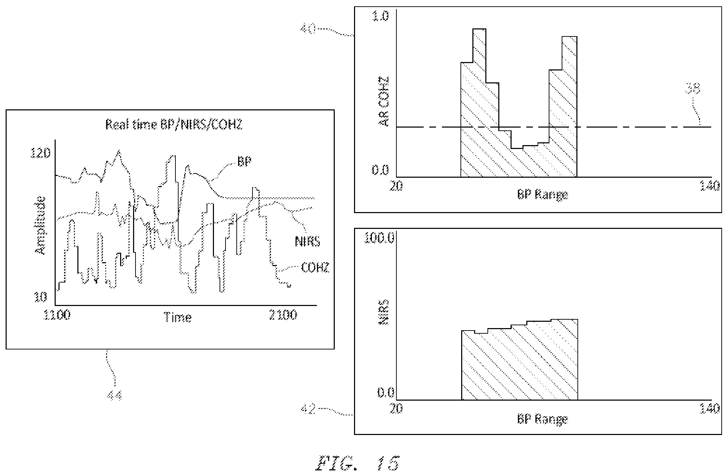

Some embodiments of the present disclosure may display one or more autoregulation plots, a short real-time window showing blood pressure and NIRS index signals and corresponding coherence signal. Some embodiments of the present disclosure may display binned values of a NIRS index as a function of blood pressure, similar to that of the autoregulation plot. The binning of a NIRS index value (e.g., a $StO_2$ value), may be triggered with at least a small change in blood pressure. A non-limiting example of a display embodiment is shown in FIG. 15, which depicts a display showing an autoregulation profile plot 40 (e.g., AR Index or COHZ versus BP Range), a corresponding plot 42 of binned NIRS index (e.g., StO2) values versus BP Range, and a real-time window 44 showing blood pressure (e.g., a mean blood pressure), a NIRS index (e.g., StO2), and COHZ as a function of time.

In some embodiments of the present disclosure, an autoregulation profile plot may reflect data for an entire monitoring period. In some embodiments, an autoregulation profile plot may reflect data collected during a period of time less than the entire monitoring period. A present disclosure AR system may be configured to selectively display either of these embodiments.

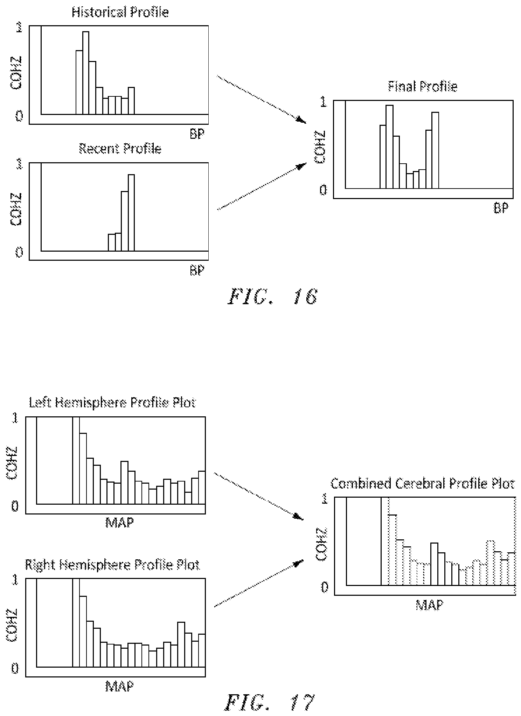

In some embodiments of the present disclosure the AM system 20 may be configured (e.g., via stored instructions) to produce both a historical autoregulation profile and a recent autoregulation profile as shown in FIG. 16. The historical autoregulation profile may include COHZ values collected over all or substantially all of the monitoring period. This period of time may be referred to as a "historical portion" of the monitoring period. An example of a historical portion that is substantially all of the monitoring period is one in which the historical period is the entire monitoring period less the recent period; e.g., if the entirety of the monitoring period is referred to as starting at a first point in time T1 and extending to a current point in time T2, a historical portion that is substantially all of the monitoring period may be described as extending between T1 and T3, where T3 is a point in time substantially later than T1 but earlier than T2, and the recent portion described as extending from T3 to T2. The recent autoregulation profile includes COHZ values collected over a recent portion of the monitoring period (e.g., the last 30 minutes) which is typically much shorter than the entire monitoring period. In both the historical portion and the recent portion of the monitoring period, the COHZ values may be determined in a manner described above; e.g., using blood pressure and NIRS index values transformed from a time domain to a frequency domain, and COHZ values determined in single frequency bands, or as otherwise described herein. The COHZ values within the historical portion or recent portion of the monitoring period may be an average (or mean, or median, etc.) of the COHZ values determined from data collected in the respective portion. In both the historical autoregulation profile and the recent autoregulation profile, the respective average COHZ values may be binned in blood pressure increments (e.g., every 5 mmHg), or in incremental blood pressure ranges (e.g., 0-20 mmHg, 20-25 mmHg, 25-30 mmHg, etc.), or the like. The average COHZ values in the historical autoregulation profile present a "smoothed" representation of the COHZ values that mitigates any collected extreme COHZ values and provides desirable COHZ trending. In addition, the historical autoregulation profile may enhance a clinician's ability to evaluate a subject's autoregulation function in response to certain events, e.g., administration of drugs, or during a surgical procedure, or the like. The COHZ values within the recent autoregulation profile, which are independent of the COHZ values in the historical autoregulation profile, provide autoregulation function data representative of the subject's current autoregulation function, and can quickly inform a clinician regarding any recent dramatic changes. The prompt knowledge of any recent autoregulation function changes can enable a clinician to take swift action should that be necessary. As shown in FIG. 16, in some embodiments the AM system 20 may be further configured (e.g., via stored instructions) to produce a compilation profile (referred to hereinafter as a "final autoregulation profile") that combines the COHZ values from the historical autoregulation profile and the recent autoregulation profile. The term "combines" as used here refers to the COHZ values from the historical autoregulation profile and the recent autoregulation profile being plotted on the same graph, or presented in the same data table, or the like. Combining the COHZ values from the historical autoregulation profile and the recent autoregulation profile does not involve mathematical addition of the COHZ values from the respective profiles. In those embodiments wherein the historical portion is the entire monitoring period less the recent portion, both the historical autoregulation profile and the final autoregulation profile may be periodically refreshed so that the COHZ values from the immediate past recent autoregulation profile are combined with the historical autoregulation profile to update the historical autoregulation profile. The updated historical autoregulation profile is then combined with the then current recent autoregulation profile to create an updated final autoregulation profile. The final autoregulation profile provides the information display advantages of both the historical autoregulation profile and the recent autoregulation profile, e.g., a "smoothed" representation of COHZ values collected over an extended period of time, COHZ trending, and prompt knowledge of any recent autoregulation function changes.

In some embodiments of the present disclosure the AM system 20 may be configured (e.g., via stored instructions) to produce both a left side cerebral autoregulation profile and a right side autoregulation profile as shown in FIG. 17. The left side autoregulation profile is based on COHZ values determined in a manner described herein, based on NIRS index data collected from a NIRS sensor applied to sense the left hemisphere of a subject's brain. The right side autoregulation profile is also based on COHZ values determined in a manner described herein, based on NIRS index data collected from a NIRS sensor applied to sense the right hemisphere of a subject's brain. The COHZ values for each side may be determined from blood pressure and NIRS index data collected over a predetermined period of time, or the COHZ values may be based on blood pressure and NIRS index data collected over an entire monitoring period, or both. As shown in FIG. 17, in some embodiments the AM system 20 may be further configured (e.g., via stored instructions) to produce a compilation profile (referred to hereinafter as a "combined sides autoregulation profile") that combines the COHZ values from the left side autoregulation profile and the right side autoregulation profile. In this embodiment, the COHZ values from the left side and right side autoregulation profiles may be combined such that the maximum COHZ ("MAX COHZ") value for each blood pressure bin is shown in the combined sides autoregulation profile. An autoregulation function profile display such as that shown in FIG. 17 is advantageous in that allows a clinician to look in one location for maximum COHZ values associated with cerebral tissue (i.e., the combined sides autoregulation profile), but also provides the clinician with autoregulation function data that is hemisphere specific. If there is a potential autoregulation function issue, the display shown in FIG. 17 will likely enable the clinician to quickly determine if the issue is unique to one brain hemisphere or common to both hemispheres.

Existing autoregulation monitoring systems may produce autoregulation function data after sensing for a predetermined period of time (e.g., 5 minutes, 10 minutes, etc.). These systems typically provide no autoregulation data function until after the aforesaid predetermined period of time, e.g., after the 5 minute period, or after the 10 minute period, etc.

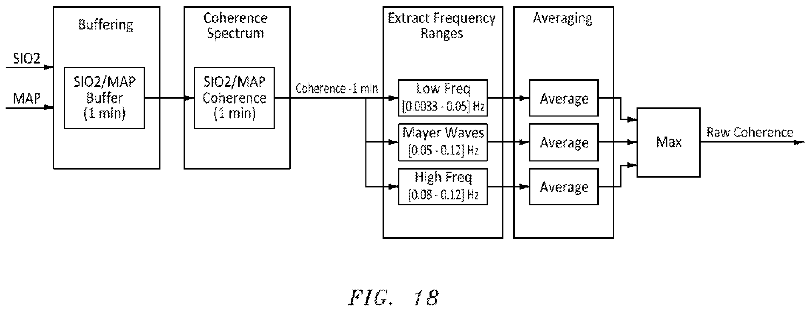

Some embodiments of the present disclosure AM system 20 may be configured (e.g., via stored instructions) to overcome this shortfall of existing systems by producing autoregulation function data almost immediately via a "start-up" autoregulation profile. The COHZ values within the startup autoregulation profile may be based on data (e.g., NIRS index and blood pressure data) collected from the start of the autoregulation monitoring. For example, the AM system 20 may be configured to produce autoregulation function data (e.g., COHZ values) based on blood pressure and NIRS index data collected during a start-up period, such as the first minute, and then produce an autoregulation profile (e.g., COHZ versus blood pressure) and/or CAI data based thereon. In the embodiment schematically shown in FIG. 17, the AM system 20 is configured to collect autoregulation function data (e.g., blood pressure and NIRS index values) during the first minute (or 80 seconds, etc.) of the monitoring period and use that data to produce COHZ values and an autoregulation profile. The embodiment shown in FIG. 18 includes steps (performed via stored instructions) wherein the COHZ values are extracted and organized in terms of a plurality of different predetermined frequency bands (e.g., a low frequency band (0.0033 - 0.05 Hz), a middle frequency band associated with Mayer waves (0.05 - 0.12 Hz); and a high frequency band (0.08 - 0.12 Hz), or other bands). The COHZ values in each frequency band are averaged, and the MAX COHZ values are subsequently used to produce a startup autoregulation function profile (e.g., MAX COHZ versus blood pressure). In another embodiment and as described above, a short sample window frequency band (e.g., COHZ frequency band #5 in FIG. 9A) may be used to produce COHZ values and those COHZ values may then be used to produce autoregulation profile and/or CAI information. The methodology described above relating to FIGS. 9 and 9A are non-limiting examples of how COHZ values in respective frequency bands may be determined, and how MAX COHZ values may be determined. In some embodiments, the AM system 20 may be configured to produce a "rolling" start-up profile. For example, the AM system may be configured to collect autoregulation function data during the first minute (or 80 seconds, etc.) of the monitoring period and use that data to produce COHZ values and an autoregulation profile. The same collection and data processing may be repeated, to produce autoregulation function data and profile for the following minute, etc., until the autoregulation data for the first predetermined period of time (e.g., 5 minutes, 10 minutes, etc.) is available. In this manner, a rolling display of the most recent autoregulation function data and profile is provided during the startup period for use by the clinician. The specific duration of the rolling display may be altered (e.g., one minute display periods, two minute display periods, etc.) by an input command to the AM system 20 to suit the needs or preferences of the end-user.

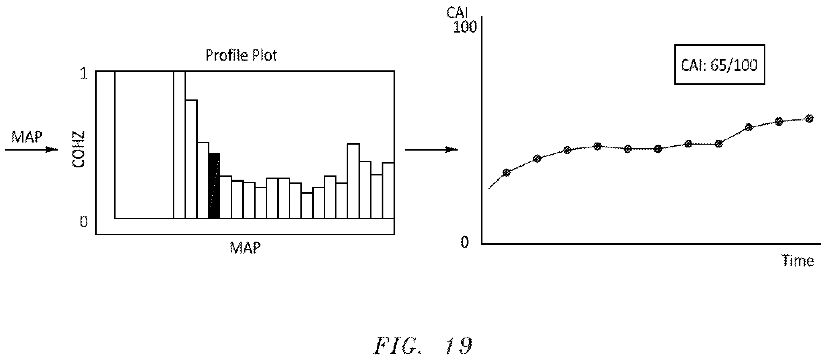

Some embodiments of the present disclosure AM system 20 may be configured (e.g., via stored instructions) to produce cerebral autoregulation index (CAI) data as a function of time. The CAI data is based at least in part on COHZ data (or correlation data) produced as described herein and organized as a function of blood pressure (e.g., MAP). In these embodiments, the AM system is configured to determine a subject blood pressure value (e.g., a MAP value). Using that determined value, the AM system selects the MAP bin aligned with the determined MAP value to identify the COHZ value associated with the selected MAP bin. That COHZ value may then be used to determine a CAI data point. FIG. 19 diagrammatically illustrates a profile plot of COHZ values versus MAP bins, and a graph of CAI values (vertical axis) versus time (horizontal axis). The CAI graph advantageously provides CAI data, as well as historical CAI data which may reveal a trend.

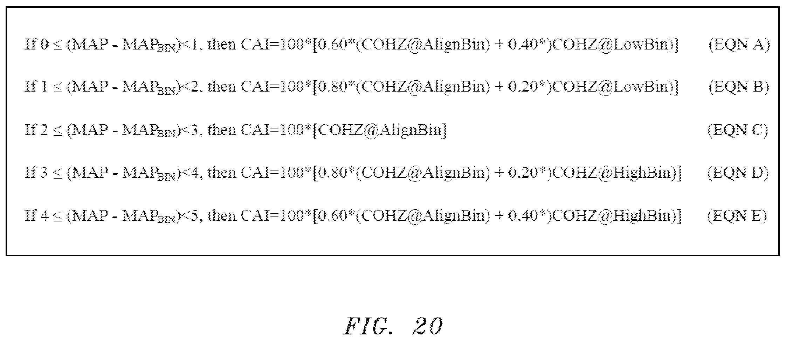

In some embodiments, the AM system 20 may be configured (e.g., via stored instructions) to produce CAI data that has been weight averaged, filtered, or otherwise processed to smooth the data and mitigate sharp differences. A non-limiting technique that can be used to smooth the CAI data involves weight averaging the COHZ value associated with the determined blood pressure value relative to the COHZ values in adjacent binned MAP values to arrive at the CAI value. For example, if the MAP values are binned in five (5) mmHg increments (e.g., 30-35 mmHg, 35-40mmHg, 40-45 mmHg, etc.), the AM system 20 may be configured to evaluate the determined MAP value relative to its position within the respective bin. In this example, each MAP bin from the profile has a 5 mmHg range, e.g., from 40 mmHg to 45 mmHg. A 41 mmHg determined MAP value may be described as "lower" in that it is closer in magnitude to the adjacent lower pressure MAP bin (i.e., 35-40 mmHg) than other values within the MAP bin, and conversely a 44 mmHg MAP value may be described as "higher" in that it is closer in magnitude to the adjacent higher pressure MAP bin (i.e., 45-50 mmHg). The COHZ value associated with determined MAP value may be weight averaged based on its magnitude position within the respective bin to arrive at the CAI value. A specific non-limited example of weight averaging may use the equations shown in FIG. 20 (assuming the MAP bin is a 5 mmHg range bin).

To illustrate, if it is assumed that the determined MAP value is 40.5 mmHg, then that determined MAP value will align with the 40-45 mmHg MAP bin. Again, for example's sake, it can be assumed further that the COHZ value of the 35-40 mmHg MAP bin (i.e., the adjacent lower pressure MAP bin) is 0.65, the COHZ value of the 40-45 mmHg MAP bin (i.e., the aligned MAP bin) is 0.55, and the COHZ value of the 45-50 mmHg MAP bin (i.e., the adjacent higher pressure MAP bin) is 0.62. To determine the weighted version of the CAI value, the determined MAP value is first evaluated relative to the aligned MAP bins follows:

$$(MAP - MAP_{BIN}) = 40.5 mmHg - 40 mmHg = 0.5$$

where the term "$MAP_{BIN}$" equals the lowest MAP value (40 mmHg) in the aligned MAP bin (40-45 mmHg). Therefore, using the equations shown in FIG. 20, EQN. A applies ($0 \le (MAP - MAP_{BIN}) < 1$), and the CAI value may be determined as follows:

$$CAI = 100 * [0.6 * (COHZ@AlignBin) + 0.4 * (COHZ@LowBin)]$$

and now with the example COHZ values entered:

$$CAI\ value = 100 * [0.6(0.55) + 0.4(0.65)] = 59.0$$

As another example, if the determined MAP value is 43.5 mmHg, then EQN. D applies ($3 \le (43.5 mmHg - 40 mmHg) < 4$), and the CAI value may be determined as follows:

$$CAI = 100 * [0.8 * (COHZ@AlignBin) + 0.2 * (COHZ@HighBin)]$$

and now with the example COHZ values entered:

$$CAI = 100 * [0.8(0.55) + 0.2(0.62)] = 56.4$$

The determined CAI values may be plotted within a CAI graph, e.g., CAI value versus time. When the subject's determined MAP is determined again (e.g., the MAP value may be determined periodically), then the above process (or other smoothing process) can be repeated to produce the latest CAI data.

As can be seen from the weighting equations shown in FIG. 20, the amount of weighting (e.g., 60%/40% versus 80%/20%) can vary depending on the magnitude of the determined MAP value relative to the MAP values within the aligned MAP bin. In addition, the example above is described in terms of MAP bins having a 5 mmHg range. The present application may be utilized with autoregulation profiles having MAP bins with a range other than 5 mmHg, and the weighting percentages may be altered to reflect the broader MAP bin range. As stated above, the above described weighting technique is an example of a weighting technique and the present disclosure is not limited thereto. Other smoothing techniques may be used alternatively. The present disclosure does not require any smoothing technique be used.

The CAI graph shown in FIG. 19 is a non-limiting example of how CAI data may be displayed within an embodiment of the present disclosure AM system 20. The present disclosure is not limited thereto.

The above described embodiments are described in terms of COHZ values determined using blood pressure and NIRS index values transformed from a time domain to a frequency domain, and COHZ values determined in single frequency bands, or as otherwise described herein. The present disclosure is not limited to COHZ values, or data produced in a frequency domain. The organization of data into a historical autoregulation profile, a recent autoregulation profile, a final autoregulation profile, a left profile, a right profile, and/or combined profile, a start-up profile, and/or CAI data, or any combination thereof, may be accomplished using data organized within a time domain and processed using a correlation technique.

As indicated above, the functionality described herein may be implemented, for example, in hardware, software tangibly embodied in a computer-readable medium, firmware, or any combination thereof. In some embodiments, at least a portion of the functionality described herein may be implemented in one or more computer programs. Each such computer program may be implemented in a computer program product tangibly embodied in non-transitory signals in a machine-readable storage device for execution by a computer processor. Method steps of the present disclosure may be performed by a computer processor executing a program tangibly embodied on a computer-readable medium to perform functions of the present disclosure by operating on input and generating output. Each computer program within the scope of the present claims below may be implemented in any programming language, such as assembly language, machine language, a high-level procedural programming language, or an object-oriented programming language. The programming language may, for example, be a compiled or interpreted programming language.

While the principles of the disclosure have been described above in connection with specific apparatuses and methods, it is to be clearly understood that this description is made only by way of example and not as limitation on the scope of the disclosure. Specific details are given in the above description to provide a thorough understanding of the embodiments. However, it is understood that the embodiments may be practiced without these specific details.

It is noted that the embodiments may be described as a process which is depicted as a flowchart, a flow diagram, a block diagram, etc. Although any one of these structures may describe the operations as a sequential process, many of the operations can be performed in parallel or concurrently. In addition, the order of the operations may be rearranged. A process may correspond to a method, a function, a procedure, a subroutine, a subprogram, etc.

The singular forms "a," "an," and "the" refer to one or more than one, unless the context clearly dictates otherwise. For example, the term "comprising a specimen" includes single or plural specimens and is considered equivalent to the phrase "comprising at least one specimen." The term "or" refers to a single element of stated alternative elements or a combination of two or more elements unless the context clearly indicates otherwise. As used herein, "comprises" means "includes." Thus, "comprising A or B," means "including A or B, or A and B," without excluding additional elements.

It is noted that various connections are set forth between elements in the present description and drawings (the contents of which are included in this disclosure by way of reference). It is noted that these connections are general and, unless specified otherwise, may be direct or indirect and that this specification is not intended to be limiting in this respect. Any reference to attached, fixed, connected or the like may include permanent, removable, temporary, partial, full and/or any other possible attachment option.

No element, component, or method step in the present disclosure is intended to be dedicated to the public regardless of whether the element, component, or method step is explicitly recited in the claims. No claim element herein is to be construed under the provisions of 35 U.S.C. 112(f) unless the element is expressly recited using the phrase "means for." As used herein, the terms "comprises", "comprising", or any other variation thereof, are intended to cover a non-exclusive inclusion, such that a process, method, article, or apparatus that comprises a list of elements does not include only those elements but may include other elements not expressly listed or inherent to such process, method, article, or apparatus.

While various inventive aspects, concepts and features of the disclosures may be described and illustrated herein as embodied in combination in the exemplary embodiments, these various aspects, concepts, and features may be used in many alternative embodiments, either individually or in various combinations and sub-combinations thereof. Unless expressly excluded herein all such combinations and sub-combinations are intended to be within the scope of the present application. Still further, while various alternative embodiments as to the various aspects, concepts, and features of the disclosures--such as alternative materials, structures, configurations, methods, devices, and components, and so on--may be described herein, such descriptions are not intended to be a complete or exhaustive list of available alternative embodiments, whether presently known or later developed. Those skilled in the art may readily adopt one or more of the inventive aspects, concepts, or features into additional embodiments and uses within the scope of the present application even if such embodiments are not expressly disclosed herein. For example, in the exemplary embodiments described above within the Detailed Description portion of the present specification, elements may be described as individual units and shown as independent of one another to facilitate the description. In alternative embodiments, such elements may be configured as combined elements.

Additionally, even though some features, concepts, or aspects of the disclosures may be described herein as being a preferred arrangement or method, such description is not intended to suggest that such feature is required or necessary unless expressly so stated. Still further, exemplary or representative values and ranges may be included to assist in understanding the present application, however, such values and ranges are not to be construed in a limiting sense and are intended to be critical values or ranges only if so expressly stated.

**Claims**

1. A method for providing information regarding a subject's autoregulation function state, comprising:

   continuously sensing a tissue region of a subject with a tissue oximeter during a period of time, the sensing producing first signals representative of at least one tissue oxygenation parameter;
   continuously measuring a blood pressure level of the subject during the period of time using a blood pressure sensing device, the measuring producing second signals representative of the blood pressure level of the subject;
   evaluating the at least one tissue oxygenation parameter using the first signals and the blood pressure level of the subject using the second signals, relative to one another, wherein the step of evaluating the at least one tissue oxygenation parameter using the first signals and the blood pressure level of the subject using the second signals, relative to one another includes:

      determining frequency domain tissue oxygen parameter values by performing a first frequency domain transformation of the first signals;
      determining frequency domain blood pressure values by performing a second frequency domain transformation of the second signals; and
      determining coherence (COHZ) values indicative of the subject's autoregulation state using the frequency domain tissue oxygen parameter values and the frequency domain blood pressure values;

   producing a recent profile of autoregulation data representative of the evaluated said at least one tissue oxygenation parameter and said blood pressure level relative to one another using said first signals and said second signals from a recent portion of the period of time;
   producing a historical profile of autoregulation data representative of the evaluated said at least one tissue oxygenation parameter and said blood pressure level relative to one another using said first signals and said second signals from a historical portion of the period of time, wherein the historical portion of the period of time is longer than the recent portion of the period of time, and the historical profile of autoregulation data is independent of the recent profile of autoregulation data, wherein the historical portion of the period of time extends less than an entirety of the period of time by an amount substantially equal to the recent portion of the period of time; and
   displaying the historical profile and the recent profile together;
   wherein both the recent profile of autoregulation data and the historical profile of autoregulation data include the COHZ values as a function of the blood pressure level.

2. The method of claim 1, wherein the period of time extends between a first point in time T1 and a second point in time T2, wherein the second point in time is later that the first point in time T1;

   wherein the recent profile of autoregulation data is produced using said first signals and said second signals from the recent portion of the period of time, the recent portion of the period of time extending between the second point in time T2 and a third point in time T3, wherein the third point in time is earlier than second point in time T2 and later than the first point in time T1; and
   wherein the historical profile of autoregulation data is produced using said first signals and said second signals from the historical portion of the period of time, the historical portion of the period of time extending between the first point in time T1 and the third point in time T3.

3. The method of claim 2, wherein the period of time extends between the first point in time T1 and a new second point in time NT2, and the new second point in time NT2 is later that the second point in time T2; and

updating the historical profile using the recent profile of autoregulation data; and

producing a new recent profile of autoregulation data representative of the evaluated said at least one tissue oxygenation parameter and said blood pressure level relative to one another using said first signals and said second signals from a new recent portion of the period of time, the new recent portion of the period of time extending between the new second point in time NT2 and the second point in time T2.

4. An apparatus (20) for providing information regarding a subject's autoregulation function state, comprising:

a near infra-red spectroscopy (NIRS) tissue oximeter (24), configured to continuously sense a tissue region of the subject;

a blood pressure sensing device (22), configured to continuously measure a blood pressure level of the subject; and

a controller (26) in communication with the NIRS tissue oximeter and the blood pressure sensing device, the controller including at least one processor and a memory device (34) configured to store instructions, the stored instructions when executed cause the controller to:

control the NIRS tissue oximeter to continuously sense a tissue region of the subject during a period of time, and to produce first signals representative of at least one tissue oxygenation parameter;

control the blood pressure sensing device to continuously measure a blood pressure level of the subject during the period of time, and to produce second signals representative of the measured blood pressure level of the subject;

evaluate the at least one tissue oxygenation parameter using the first signals and the blood pressure level of the subject using the second signals, relative to one another, wherein evaluating the at least one tissue oxygenation parameter using the first signals and the blood pressure level of the subject using the second signals, relative to one another includes:

determining frequency domain tissue oxygen parameter values by performing a first frequency domain transformation of the first signals;

determining frequency domain blood pressure values by performing a second frequency domain transformation of the second signals; and

determining coherence (COHZ) values indicative of the subject's autoregulation state using the frequency domain tissue oxygen parameter values and the frequency domain blood pressure values;

produce a recent profile of autoregulation data representative of the evaluated said at least one tissue oxygenation parameter and said blood pressure level relative to one another using said first signals and said second signals from a recent portion of the period of time;

produce a historical profile of autoregulation data representative of the evaluated said at least one tissue oxygenation parameter and said blood pressure level relative to one another using said first signals and said second signals from a historical portion of the period of time, wherein the historical portion of the period of time is longer than the recent portion of the period of time, and the historical profile of autoregulation data is independent of the recent profile of autoregulation data, wherein the historical portion of the period of time extends less than an entirety of the period of time by an amount substantially equal to the recent portion of the period of time; and

display the historical profile and the recent profile together;

wherein both the recent profile of autoregulation data and the historical profile of autoregulation data include the COHZ values as a function of the blood pressure level.

5. A non-transitory computer-readable medium containing computer program instructions, wherein the computer program instructions are executable by the at least one computer processor to perform a method of providing information regarding a subject's autoregulation function state, the method comprising:

controlling a near infra-red spectroscopy (NIRS) tissue oximeter (24) to continuously sense a tissue region of a subject during a period of time, the sensing producing first signals representative of at least one tissue oxygenation parameter;

controlling a blood pressure sensing device (22) to continuously measure a blood pressure level of the subject during the period of time, the measuring producing second signals representative of the blood pressure level of the subject;

evaluating the at least one tissue oxygenation parameter using the first signals and the blood pressure level of the subject using the second signals, relative to one another, wherein the step of evaluating the at least one tissue oxygenation parameter using the first signals and the blood pressure level of the subject using the second signals, relative to one another includes:

determining frequency domain tissue oxygen parameter values by performing a first frequency domain transformation of the first signals;
determining frequency domain blood pressure values by performing a second frequency domain transformation of the second signals; and
determining coherence (COHZ) values indicative of the subject's autoregulation state using the frequency domain tissue oxygen parameter

values and the frequency domain blood pressure values;

producing a recent profile of autoregulation data representative of the evaluated said at least one tissue oxygenation parameter and said blood pressure level relative to one another using said first signals and said second signals from a recent portion of the period of time;
producing a historical profile of autoregulation data representative of the evaluated said at least one tissue oxygenation parameter and said blood pressure level relative to one another using said first signals and said second signals from a historical portion of the period of time, wherein the historical portion of the period of time is longer than the recent portion of the period of time, and the historical profile of autoregulation data is independent of the recent profile of autoregulation data, wherein the historical portion of the period of time extends less than an entirety of the period of time by an amount substantially equal to the recent portion of the period of time; and
displaying the historical profile and the recent profile together;
wherein both the recent profile of autoregulation data and the historical profile of autoregulation data include the COHZ values as a function of the blood pressure level.

## Patentansprüche

1. Verfahren zum Bereitstellen von Informationen zum Autoregulationsfunktionszustand eines Subjekts, umfassend:

kontinuierliches Abfühlen einer Geweberegion eines Subjekts mit einem Gewebeoximeter während eines Zeitraums, wobei das Abfühlen erste Signale produziert, die für wenigstens einen Gewebeoxygenierungsparameter repräsentativ sind;
kontinuierliches Messen eines Blutdruckniveaus des Subjekts während des Zeitraums unter Verwendung einer Blutdruckabfühlvorrichtung, wobei die Messung zweite Signale produziert, die für das Blutdruckniveau des Subjekts repräsentativ sind;
Auswerten des wenigstens einen Gewebeoxygenierungsparameters unter Verwendung der ersten Signale und des Blutdruckniveaus des Subjekts unter Verwendung der zweiten Signale, relativ zueinander, wobei der Schritt des Auswertens des wenigstens einen Gewebeoxygenierungsparameters unter Verwendung der ersten Signale und des Blutdruckniveaus des Subjekts unter Verwendung der zweiten Signale, relativ zueinander, dies beinhaltet:

Bestimmen von Frequenzdomänenwerten des Gewebeoxygenierungsparameters durch Durchführen einer ersten Frequenzdomänentransformation der ersten Signale;
Bestimmen von Frequenzdomänenwerten des Blutdrucks durch Durchführen einer zweiten Frequenzdomänentransformation der zweiten Signale; und
Bestimmen von Kohärenz (COHZ)-Werten, die den Autoregulationszustand des Subjekts unter Verwendung der Frequenzdomänenwerte des Gewebeoxygenierungsparameters und der Frequenzdomänenwerte des Blutdrucks angeben;
Produzieren eines aktuellen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem aktuellen Abschnitt des Zeitraums;
Produzieren eines historischen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem historischen Abschnitt des Zeitraums, wobei der historische Abschnitt des Zeitraums länger als der aktuelle Abschnitt des Zeitraums ist und das historische Profil von Autoregulationsdaten unabhängig von dem aktuellen Profil von Autoregulationsdaten ist, wobei sich der historische Abschnitt des Zeitraums über weniger als eine Gesamtheit des Zeitraums erstreckt, um eine Menge, die im Wesentlichen gleich dem aktuellen Abschnitt des Zeitraums ist; und

gemeinsames Anzeigen des historischen Profils und des aktuellen Profils;

wobei sowohl das aktuelle Profil von Autoregulationsdaten als auch das historische Profil von Autoregulationsdaten die COHZ-Werte in Abhängigkeit von dem Blutdruckniveau beinhalten.

2. Verfahren nach Anspruch 1, wobei sich der Zeitraum zwischen einem ersten Zeitpunkt T1 und einem zweiten Zeitpunkt T2 erstreckt, wobei der zweite Zeitpunkt nach dem ersten Zeitpunkt T1 liegt;

wobei das aktuelle Profil von Autoregulationsdaten unter Verwendung der ersten Signale und der zweiten Signale aus dem aktuellen Abschnitt des Zeitraums produziert wird und sich der aktuelle Abschnitt des Zeitraums zwischen dem zweiten Zeitpunkt T2 und einem dritten Zeitpunkt T3 erstreckt, wobei der dritte Zeitpunkt vor dem zweiten Zeitpunkt T2 und nach dem ersten Zeitpunkt T1 liegt; und wobei das historische Profil von Autoregulationsdaten unter Verwendung der ersten Signale und der zweiten Signale aus dem historischen Abschnitt des Zeitraums produziert wird, wobei sich der historische Abschnitt des Zeitraums zwischen dem ersten Zeitpunkt T1 und dem dritten Zeitpunkt T3 erstreckt.

3. Verfahren nach Anspruch 2, wobei sich der Zeitraum zwischen dem ersten Zeitpunkt T1 und einen neuen zweiten Zeitpunkt NT2 erstreckt und der neue zweite Zeitpunkt NT2 nach dem zweiten Zeitpunkt T2 liegt; und

Aktualisieren des historischen Profils unter Verwendung des aktuellen Profils von Autoregulationsdaten; und Produzieren eines neuen aktuellen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem neuen aktuellen Abschnitt des Zeitraums, wobei sich der neue aktuelle Abschnitt des Zeitraums zwischen dem neuen zweiten Zeitpunkt NT2 und dem zweiten Zeitpunkt T2 erstreckt.

4. Einrichtung (20) zum Bereitstellen von Informationen zum Autoregulationsfunktionszustand eines Subjekts, umfassend:

Nah-Infrarot-Spektroskopie (NIRS)-Gewebeoximeter (24), das dazu ausgelegt ist, eine Geweberegion des Subjekts kontinuierlich abzufühlen;

eine Blutdruckabfühlvorrichtung (22), die dazu ausgelegt ist, ein Blutdruckniveau des Subjekts kontinuierlich zu messen; und

eine Steuerung (26) in Kommunikation mit dem NIRS-Gewebeoximeter und der Blutdruckabfühlvorrichtung, wobei die Steuerung wenigstens einen Prozessor und eine Speichervorrichtung (34) einschließt, die dazu ausgelegt ist, Anweisungen zu speichern, wobei die gespeicherten Anweisungen bei Ausführung bewirken, dass die Steuerung folgendes ausführt:

Steuern des NIRS-Gewebeoximeters zum kontinuierlichen Abfühlen einer Geweberegion des Subjekts während eines Zeitraums und zum Produzieren von ersten Signalen, die wenigstens einen Gewebeoxygenierungsparameter repräsentieren;

Steuern der Blutdruckabfühlvorrichtung, um ein Blutdruckniveau des Subjekts während des Zeitraums kontinuierlich zu messen und zweite Signale zu produzieren, die für das gemessene Blutdruckniveau des Subjekts repräsentativ sind;

Auswerten des wenigstens einen Gewebeoxygenierungsparameters unter Verwendung der ersten Signale und des Blutdruckniveaus des Subjekts unter Verwendung der zweiten Signale, relativ zueinander,

wobei das Auswerten des wenigstens einen Gewebeoxygenierungsparameters unter Verwendung der ersten Signale und des Blutdruckniveaus des Subjekts unter Verwendung der zweiten Signale, relativ zueinander, dies beinhaltet:

Bestimmen von Frequenzdomänenwerten des Gewebeoxygenierungsparameters durch Durchführen einer ersten Frequenzdomänentransformation der ersten Signale;

Bestimmen von Frequenzdomänenwerten des Blutdrucks durch Durchführen einer zweiten Frequenzdomänentransformation der zweiten Signale; und

Bestimmen von Kohärenz (COHZ)-Werten, die den Autoregulationszustand des Subjekts unter Verwendung der Frequenzdomänenwerte des Gewebeoxygenierungsparameters und der Frequenzdomänenwerte des Blutdrucks angeben;

Produzieren eines aktuellen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem aktuellen Abschnitt des Zeitraums;

Produzieren eines historischen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem historischen Abschnitt des Zeitraums, wobei der historische Abschnitt des Zeitraums länger als der aktuelle Abschnitt des Zeitraums ist und das historische Profil von Autoregulationsdaten unabhängig von dem aktuellen Profil von Autoregulationsdaten ist, wobei sich der historische Abschnitt des Zeitraums über weniger als eine Gesamtheit des Zeitraums erstreckt, um eine Menge, die im Wesentlichen gleich dem aktuellen Abschnitt des Zeitraums ist; und

gemeinsames Anzeigen des historischen Profils und des aktuellen Profils;

wobei sowohl das aktuelle Profil von Autoregulationsdaten als auch das historische Profil von Autoregulationsdaten die COHZ-Werte in Abhängigkeit von dem Blutdruckniveau beinhalten.

5. Nicht-transitorisches computerlesbares Medium mit Computerprogrammanweisungen, wobei die Computerprogrammanweisungen von dem wenigstens einen Computerprozessor ausgeführt werden können, um ein Verfahren zum Bereitstellen von Informationen zum Autoregulationsfunktionszustand eines Subjekts durchzuführen, wobei das Verfahren umfasst:

ein Nah-Infrarot-Spektroskopie (NIRS)-Gewebeoximeter (24), das dazu ausgelegt ist, eine Geweberegion eines Subjekts während eines Zeitraums kontinuierlich abzufühlen, wobei das Abfühlen erste Signale produziert, die für wenigstens einen Gewebeoxygenierungsparameter repräsentativ sind;

Steuern einer Blutdruckabfühlvorrichtung (22), um ein Blutdruckniveau des Subjekts während des Zeitraums kontinuierlich zu messen, wobei die Messung zweite Signale produziert, die für das Blutdruckniveau des Subjekts repräsentativ sind;

Auswerten des wenigstens einen Gewebeoxygenierungsparameters unter Verwendung der ersten Signale und des Blutdruckniveaus des Subjekts unter Verwendung der zweiten Signale, relativ zueinander, wobei der Schritt des Auswertens des wenigstens einen Gewebeoxygenierungsparameters unter Verwendung der ersten Signale und des Blutdruckniveaus des Subjekts unter Verwendung der zweiten Signale, relativ zueinander, dies beinhaltet:

Bestimmen von Frequenzdomänenwerten des Gewebeoxygenierungsparameters durch Durchführen einer ersten Frequenzdomänentransformation der ersten Signale;

Bestimmen von Frequenzdomänenwerten des Blutdrucks durch Durchführen einer zweiten Frequenzdomänentransformation der zweiten Signale; und

Bestimmen von Kohärenz (COHZ)-Werten, die den Autoregulationszustand des Subjekts unter Verwendung der Frequenzdomänenwerte des Gewebeoxygenierungsparameters und der Frequenzdomänenwerte des Blutdrucks angeben;

Produzieren eines aktuellen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem aktuellen Abschnitt des Zeitraums;

Produzieren eines historischen Profils von Autoregulationsdaten, die für den ausgewerteten wenigstens einen Gewebeoxygenierungsparameter und das Blutdruckniveau relativ zueinander repräsentativ sind, unter Verwendung der ersten Signale und der zweiten Signale aus einem historischen Abschnitt des Zeitraums, wobei der historische Abschnitt des Zeitraums länger als der aktuelle Abschnitt des Zeitraums ist und das historische Profil von Autoregulationsdaten unabhängig von dem aktuellen Profil von Autoregulationsdaten ist, wobei sich der historische Abschnitt des Zeitraums über weniger als eine Gesamtheit des Zeitraums erstreckt, um eine Menge, die im Wesentlichen gleich dem aktuellen Abschnitt des Zeitraums ist; und

gemeinsames Anzeigen des historischen Profils und des aktuellen Profils;

wobei sowohl das aktuelle Profil von Autoregulationsdaten als auch das historische Profil von Autoregulationsdaten die COHZ-Werte in Abhängigkeit von dem Blutdruckniveau beinhalten.

**Revendications**

1. Procédé de fourniture d'informations concernant un état de fonction d'autorégulation d'un sujet, comprenant :

la détection en continu d'une région tissulaire d'un sujet avec un oxymètre tissulaire pendant une période de temps, la détection produisant des premiers signaux représentatifs d'au moins un paramètre d'oxygénation tissulaire ;

la mesure en continu d'un niveau de pression sanguine du sujet pendant la période de temps au moyen d'un dispositif de détection de pression sanguine, la mesure produisant des deuxièmes signaux représentatifs du niveau de pression sanguine du sujet ;

l'évaluation de l'au moins un paramètre d'oxygénation tissulaire à l'aide des premiers signaux et du niveau de pression sanguine du sujet à l'aide des deuxièmes signaux, l'un par rapport à l'autre, l'étape d'évaluation de l'au moins un paramètre d'oxygénation tissulaire à l'aide des premiers signaux et du niveau de pression sanguine du sujet à l'aide des deuxièmes signaux, l'un par rapport à l'autre comprenant :

la détermination de valeurs de paramètres de l'oxygène tissulaire dans le domaine fréquentiel en effectuant une première transformation dans le domaine fréquentiel des premiers signaux ;

la détermination de valeurs de pression sanguine dans le domaine fréquentiel en effectuant une seconde transformation dans le domaine fréquentiel des deuxièmes signaux ; et

la détermination de valeurs de cohérence (COHZ) indicatives de l'état d'autorégulation du sujet à l'aide des valeurs de paramètres de l'oxygène tissulaire dans le domaine fréquentiel et des valeurs de la pression sanguine dans le domaine fréquentiel ;

la production d'un profil récent de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux à partir d'une partie récente de la période de temps ;

la production d'un profil historique de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux à partir d'une partie historique de la période de temps, la partie historique de la période de temps étant supérieure à la partie récente de la période de temps, et le profil historique de données d'autorégulation étant indépendant du profil récent de données d'autorégulation, la partie historique de la période de temps s'étendant moins qu'une totalité de la période de temps d'une quantité sensiblement égale à la partie récente de la période de temps ; et

l'affichage ensemble du profil historique et du profil récent ;

le profil récent de données d'autorégulation et le profil historique de données d'autorégulation comprenant tous deux les valeurs de COHZ en fonction du niveau de pression sanguine.

2. Procédé selon la revendication 1, la période de temps s'étendant entre un premier point dans le temps T1 et un deuxième point dans le temps T2, le deuxième point dans le temps étant postérieur au premier point dans le temps T1 ;

le profil récent de données d'autorégulation étant réalisé en utilisant lesdits premiers signaux et lesdits deuxièmes signaux à partir de la partie récente de la période de temps, la partie récente de la période de temps s'étendant entre le deuxième point dans le temps T2 et un troisième point dans le temps T3, le troisième point dans le temps étant antérieur au deuxième point dans le temps T2 et postérieur au premier point dans le temps T1 ; et

le profil historique de données d'autorégulation étant produit en utilisant lesdits premiers signaux et lesdits deuxièmes signaux de la partie historique de la période temporelle, la partie historique de la période temporelle s'étendant entre le premier point dans le temps T1 et le troisième point dans le temps T3.

3. Procédé selon la revendication 2, la période de temps s'étendant entre le premier point dans le temps T1 et un nouveau deuxième point dans le temps NT2, et le nouveau deuxième point dans le temps NT2 étant postérieur au deuxième point dans le temps T2 ; et

la mise à jour du profil historique à l'aide du profil récent des données d'autorégulation ; et

la réalisation d'un nouveau profil récent de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux d'une nouvelle partie récente de la période de temps, la nouvelle partie récente de la période de temps s'étendant entre le nouveau deuxième point dans le temps NT2 et le deuxième point dans le temps T2.

4. Appareil (20) pour fournir des informations concernant un état de fonction d'autorégulation d'un sujet, comprenant :

un oxymètre tissulaire (24) de spectroscopie proche infrarouge (NIRS), configuré pour détecter en continu une région tissulaire du sujet ;
un dispositif de détection de pression sanguine (22) conçu pour mesurer en continu un niveau de pression sanguine du sujet ; et
un dispositif de commande (26) en communication avec l'oxymètre tissulaire NIRS et le dispositif de détection de la pression sanguine, le dispositif de commande comportant au moins un processeur et un dispositif de mémoire (34) configuré pour stocker des instructions, les instructions stockées lorsqu'elles sont exécutées amenant le dispositif de commande à :

commander l'oxymètre tissulaire NIRS pour détecter en continu une région tissulaire du sujet pendant une période de temps, et pour produire des premiers signaux représentatifs d'au moins un paramètre d'oxygénation tissulaire ;
commander le dispositif de détection de la pression sanguine afin de mesurer en continu un niveau de pression sanguine du sujet pendant la période de temps, et de produire des deuxièmes signaux représentatifs du niveau de pression sanguine du sujet ;
évaluer l'au moins un paramètre d'oxygénation tissulaire au moyen des premiers signaux et du niveau de pression sanguine du sujet au moyen des deuxièmes signaux, l'un par rapport à l'autre, l'évaluation de l'au moins un paramètre d'oxygénation tissulaire au moyen des premiers signaux et du niveau de pression sanguine du sujet au moyen des deuxièmes signaux, l'un par rapport à l'autre comprenant :

la détermination de valeurs de paramètres de l'oxygène tissulaire dans le domaine fréquentiel en effectuant une première transformation dans le domaine fréquentiel des premiers signaux ;
la détermination de valeurs de pression sanguine dans le domaine fréquentiel en effectuant une seconde transformation dans le domaine fréquentiel des deuxièmes signaux ; et
la détermination de valeurs de cohérence (COHZ) indicatives de l'état d'autorégulation du sujet à l'aide des valeurs de paramètres de l'oxygène tissulaire dans le domaine fréquentiel et des valeurs de la pression sanguine dans le domaine fréquentiel ;
produire un profil récent de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux à partir d'une partie récente de la période de temps ;
produire un profil historique de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux à partir d'une partie historique de la période de temps, la partie historique de la période de temps étant supérieure à la partie récente de la période de temps, et le profil historique de données d'autorégulation étant indépendant du profil récent de données d'autorégulation, la partie historique de la période de temps s'étendant moins qu'une totalité de la période de temps d'une quantité sensiblement égale à la partie récente de la période de temps ; et
afficher ensemble le profil historique et le profil récent ;
le profil récent de données d'autorégulation et le profil historique de données d'autorégulation comprenant tous deux les valeurs de COHZ en fonction du niveau de pression sanguine.

5. Support lisible par ordinateur non transitoire contenant des instructions de programme informatique, les instructions de programme informatique étant exécutables par l'au moins un processeur informatique pour réaliser un procédé de fourniture d'informations concernant l'état de fonction d'autorégulation d'un sujet, le procédé comprenant :

la commande d'un oxymètre tissulaire (24) de spectroscopie proche infrarouge (NIRS), pour détecter en continu une région tissulaire d'un sujet pendant une période de temps, la détection produisant des premiers signaux représentatifs d'au moins un paramètre d'oxygénation tissulaire ;
la commande du dispositif de détection de la pression sanguine (22) afin de mesurer en continu un niveau de pression sanguine du sujet pendant la période de temps,
la mesure produisant des deuxièmes signaux représentatifs de la pression sanguine du sujet ;
l'évaluation de l'au moins un paramètre d'oxygénation tissulaire à l'aide des premiers signaux et du niveau de pression sanguine du sujet à l'aide des deuxièmes signaux, l'un par rapport à l'autre, l'étape d'évaluation de l'au moins un paramètre d'oxygénation tissulaire à l'aide des premiers signaux et du niveau de pression sanguine du sujet à l'aide des deuxièmes signaux, l'un par rapport à l'autre comprenant :

la détermination de valeurs de paramètres de l'oxygène tissulaire dans le domaine fréquentiel en effectuant une première transformation dans le domaine fréquentiel des premiers signaux ;

la détermination de valeurs de pression sanguine dans le domaine fréquentiel en effectuant une seconde transformation dans le domaine fréquentiel des deuxièmes signaux ; et

la détermination de valeurs de cohérence (COHZ) indicatives de l'état d'autorégulation du sujet à l'aide des valeurs de paramètres de l'oxygène tissulaire dans le domaine fréquentiel et des valeurs de la pression sanguine dans le domaine fréquentiel ;

la production d'un profil récent de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux à partir d'une partie récente de la période de temps ;

la production d'un profil historique de données d'autorégulation représentatives dudit au moins un paramètre d'oxygénation tissulaire et dudit niveau de pression sanguine évalués l'un par rapport à l'autre à l'aide desdits premiers signaux et desdits deuxièmes signaux à partir d'une partie historique de la période de temps, la partie historique de la période de temps étant supérieure à la partie récente de la période de temps, et le profil historique de données d'autorégulation étant indépendant du profil récent de données d'autorégulation, la partie historique de la période de temps s'étendant moins qu'une totalité de la période de temps d'une quantité sensiblement égale à la partie récente de la période de temps ; et

l'affichage ensemble du profil historique et du profil récent ;

le profil récent de données d'autorégulation et le profil historique de données d'autorégulation comprenant tous deux les valeurs de COHZ en fonction du niveau de pression sanguine.

FIG. 1
(PRIOR ART)

FIG. 2
(PRIOR ART)

**FIG. 3**
(PRIOR ART)

**FIG. 4A**

FIG. 4B

FIG. 5

*FIG. 6*

FIG. 7

EP 4 304 462 B1

*FIG. 8*

COHZ FREQUENCY BAND #1:
(e.g., 0.00333-0.05 Hz)
5 minute Sampling Window

COHZ FREQUENCY BAND #2:
(e.g., 0.00166-0.05 Hz)
10 minute Sampling Window

COHZ FREQUENCY BAND #3:
(e.g., 0.000833-0.05 Hz)
20 minute Sampling Window

COHZ FREQUENCY BAND #4:
(e.g., 0.05-0.15 Hz)
5 minute Sampling Window

COHZ FREQUENCY BAND #5
(e.g., 0.08-0.12 Hz) - Mayer Wave
5 minute Sampling Window

COHZ Peak
Detector

36

AR Index:
Determined
MAX COHZ

FIG. 9

COHZ FREQUENCY BAND #1:
(very slow ΔBP changes – Very low ƒ)
(e.g., 0.00166-0.0033 Hz)
10 Minute Sampling Window

COHZ FREQUENCY BAND #2:
(slow ΔBP changes – low ƒ-1 )
(e.g., 0.0034-0.011667 Hz)
5 Minute Sampling Window

COHZ FREQUENCY BAND #3:
(slow ΔBP changes – low ƒ-2 )
(e.g., 0.0125-0.020833 Hz)
5 Minute Sampling Window

COHZ FREQUENCY BAND #4:
(slow ΔBP changes – low ƒ-3 )
(e.g., 0.021667-0.03 Hz)
5 Minute Sampling Window

COHZ FREQUENCY BAND #5:
(Fast Startup – low ƒ )
(e.g., 0.0125-0.03 Hz)
80 Second Sampling Window

COHZ Peak
Detector

36

AR Index:
Determined
MAX COHZ

**FIG. 9A**

COHZ FREQUENCY
BAND #1:
(e.g., 0.00333-0.05 Hz)
5 minute Sampling Window

COHZ FREQUENCY BAND #2:
(e.g., 0.00166-0.05 Hz)
10 minute Sampling Window

COHZ FREQUENCY BAND #5:
(e.g., 0.06-0.12 Hz)
- Mayer Wave
5 minute Sampling Window

COHZ FREQUENCY BAND #3:
(e.g., 0.000833-0.05 Hz)
20 minute Sampling Window

COHZ FREQUENCY BAND #4:
(e.g., 0.05-0.15 Hz)
5 minute Sampling Window

0.000833 Hz          0.05 Hz          0.15 Hz

FREQUENCY

*FIG. 10*

COHZ FREQ. BAND #5:
(e.g., 0.0125-0.03 Hz)
80 Sec Sampling Window

COHZ FREQUENCY BAND #4:
(e.g., 0.021667-0.03 Hz)
5 Minute Sampling Window

COHZ FREQUENCY BAND #3:
(e.g., 0.0125-0.020833 Hz)
5 Minute Sampling Window

COHZ FREQUENCY BAND #2:
(e.g., 0.0034-0.011667 Hz)
5 Minute Sampling Window

COHZ FREQUENCY BAND #1:
(e.g., 0.00166-0.0033 Hz)
10 Minute Sampling Window

0.00166 Hz          0.0034 Hz          0.0125 Hz          0.03 Hz

FREQUENCY

**FIG. 10A**

COHZ FREQUENCY
BAND #4
(e.g., 0.05-0.15 Hz)
5 minute Sampling
Window

COHZ FREQUENCY
BAND #5
(e.g., 0.08-0.12 Hz)
Mayer Wave
5 minute Sampling
Window

COHZ FREQUENCY
BAND #1
(e.g., 0.00833-0.05 Hz)
5 minute Sampling
Window

COHZ FREQUENCY BAND #2
(e.g., 0.00166-0.05 Hz)
10 minute Sampling Window

COHZ FREQUENCY BAND #3
(e.g., 0.000833-0.05 Hz)
20 minute Sampling Window

$T_{-20mins}$  $T_{-10mins}$  $T_{-5mins}$  $T_{PRESENT}$

TIME

FIG. 11

FIG. 11A

FIG. 12

FIG. 12A

FIG. 13

FIG. 14

*FIG. 15*

Historical Profile

Recent Profile

Final Profile

FIG. 16

Left Hemisphere Profile Plot

Right Hemisphere Profile Plot

Combined Cerebral Profile Plot

FIG. 17

*FIG. 18*

*FIG. 19*

If $0 \leq$ (MAP - $MAP_{BIN}$)<1, then CAI=100*[0.60*(COHZ@AlignBin) + 0.40*)COHZ@LowBin)]     (EQN A)

If $1 \leq$ (MAP - $MAP_{BIN}$)<2, then CAI=100*[0.80*(COHZ@AlignBin) + 0.20*)COHZ@LowBin)]     (EQN B)

If $2 \leq$ (MAP - $MAP_{BIN}$)<3, then CAI=100*[COHZ@AlignBin]     (EQN C)

If $3 \leq$ (MAP - $MAP_{BIN}$)<4, then CAI=100*[0.80*(COHZ@AlignBin) + 0.20*)COHZ@HighBin)]     (EQN D)

If $4 \leq$ (MAP - $MAP_{BIN}$)<5, then CAI=100*[0.60*(COHZ@AlignBin) + 0.40*)COHZ@HighBin)]     (EQN E)

*FIG. 20*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63181108
- US 2020383616 A
- US 2020129076 A
- US 2017105672 A
- US 2021016136 A
- US 6456862 B
- US 7072701 B
- US 8078250 B
- US 8396526 B
- US 8965472 B
- US 10117610 B
- US 9913601 B
- US 9848808 B
- US 9456773 B
- US 9364175 B
- US 9923943 B
- US 8788004 B